# EUROPÄISCHE PATENTANMELDUNG

(11) **EP 0 678 514 A1**
(43) Veröffentlichungstag der Anmeldung: **25.10.1995**
(21) Anmeldenummer: 95810237.8
(22) Anmeldetag: 07.04.1995
(51) Int. Cl.: C07D 307/32, C07D 263/26, C07D 413/06, C07D 295/12, C07C 237/20

(54) **3,5-Disubstituierte Tetrahydrofuran-2-one**

(30) Priorität: 18.04.1994 CH 1169/94; 30.01.1995 CH 246/95
(71) Anmelder: CIBA-GEIGY AG, CH-4002 Basel (CH)
(72) Erfinder: Göschke, Richard, Dr., CH-4103 CH-Bottmingen (DE); Herold, Peter, Dr., CH-4144 Arlesheim (CH); Rigollier, Pascal, Dr., F-68510 Sierentz (FR); Maibaum, Jürgen Klaus, Dr., D-79576 Weil-Haltingen (DE)

(57) **Zusammenfassung**

Verbindungen der Formel I 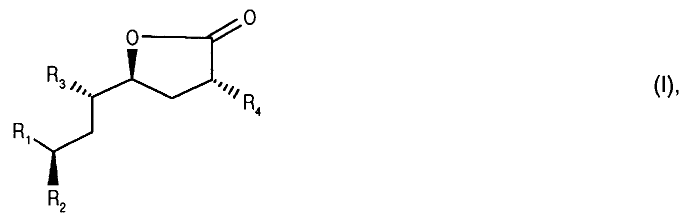
worin R₁ gegebenenfalls verestertes Carboxy, Hydroxymethyl oder Formyl darstellt, R₂ und R₄ unabhängig voneinander aliphatische, cycloaliphatisch-aliphatische, araliphatische oder heteroarylaliphatische Reste bedeuten und R₃ Azido oder aliphatisch oder araliphatisch substituiertes und/oder durch eine Aminoschutzgruppe geschütztes Amino darstellt, und ihre Salze sind wertvolle Zwischenprodukte für die Herstellung von Arzneimittelwirkstoffen, beispielsweise von Verbindungen der Formel II 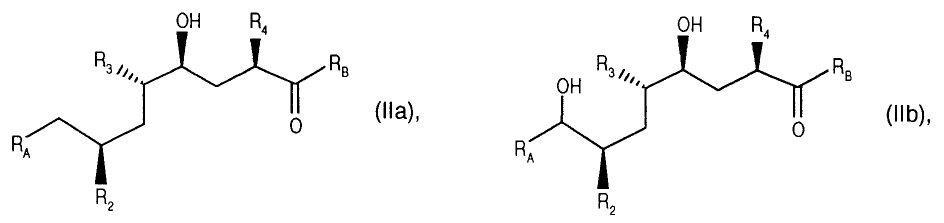
worin R_{A} eine aromatischen oder heteroaromatischen Rest bedeutet, R₂ und R₄ unabhängig voneinander aliphatische, cycloaliphatische, cycloaliphatisch-aliphatische oder araliphatische Reste darstellen, R₃ für unsubstituiertes oder N-mono- oder N,N-diniederalkyliertes oder N-niederalkanoyliertes Amino steht und R_{B} eine aliphatisch, cycloaliphatisch oder heteroaromatisch-aliphatisch substituierte Aminogruppe bedeutet, und ihrer Salze, die beispielsweise als Antihypertensive eingesetzt werden können.

## Beschreibung

Die Erfindung betrifft Verbindungen der Formel I 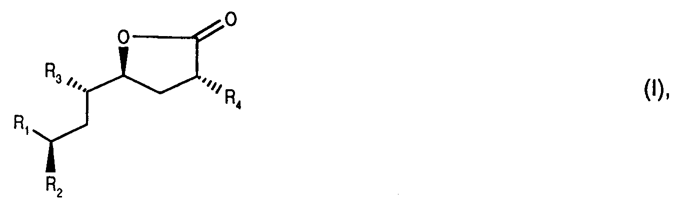
worin
R₁ gegebenenfalls verestertes Carboxy, Hydroxymethyl oder Formyl darstellt,
R₂ und R₄ unabhängig voneinander aliphatische, cycloaliphatisch-aliphatische, araliphatische oder heteroarylaliphatische Reste bedeuten und
R₃ Azido oder aliphatisch oder araliphatisch substituiertes und/oder durch eine Aminoschutzgruppe geschütztes Amino darstellt,
und ihre Salze, Verfahren zu ihrer Herstellung und ihre Verwendung als Zwischenprodukte für die Herstellung von Arzneimittelwirkstoffen.

Gegebenenfalls verestertes Carboxy ist beispielsweise Carboxy, Niederalkoxycarbonyl oder unsubstituiertes oder durch Niederalkyl, Niederalkoxy, Halogen und/oder Trifluormethyl substituiertes Phenylniederalkoxycarbonyl.

Aliphatische Reste sind beispielsweise Niederalkylreste, Niederalkenyl, Niederalkoxy oder Niederalkylthio, vorzugsweise Methyl oder verzweigtes C₁-C₄-Alkyl, wie Isopropyl.

Cycloaliphatisch-aliphatische Reste sind beispielsweise 3- bis 7, insbesondere 3- bis 5-gliedrige Cycloalkylniederalkylreste.

Araliphatische Reste sind beispielsweise unsubstituiertes oder durch Niederalkyl, Niederalkoxy, Hydroxy, Niederalkylamino, Diniederalkylamino, Halogen und/oder Trifluormethyl mono-, di- oder trisubstituiertes Arylniederalkyl, wie Phenylniederalkyl oder Naphthylniederalkyl.

Heteroarylaliphatische Reste weisen als Heteroarylrest beispielsweise einen gegebenenfalls benzokondensierten 5- oder 6-gliedrigen Mono- oder Diazaarylrest oder 5-gliedrigen Oxa- oder Thiaarylrest auf und bedeuten beispielsweise unsubstituiertes oder durch Niederalkyl, Niederalkoxy, Hydroxy, Halogen, Nitro und/oder Amino substituiertes Pyridylniederalkyl, Indolylniederalkyl, Chinolinylniederalkyl, Pyrimidinylniederalkyl, Furylniederalkyl, Benzofuranylniederalkyl oder Thienylniederalkyl.

Unsubstituiertes oder aliphatisch oder araliphatisch substituiertes Amino ist beispielsweise gegebenenfalls aliphatisch mono- oder disubstituiertes, araliphatisch monosubstituiertes oder araliphatisch und aliphatisch disubstitutiertes Amino, wie Niederalkylamino, Diniederalkylamino, Niederalkanoylamino oder N-Niederalkanoyl-N-niederalkyl-amino, unsubstituiertes oder durch Niederalkyl, Niederalkoxy, Hydroxy, Halogen, Nitro und/oder Amino substituiertes Phenylniederalkylamino, N-Niederalkyl-N-phenylniederalkyl-amino oder N-Niederalkanoyl-N-phenylniederalkyl-amino.

Durch eine Aminoschutzgruppe geschütztes aliphatisch oder araliphatisch substituiertes Amino ist beispielsweise durch eine von einem Halbester der Kohlensäure oder einer aromatischen Carbonsäure abgeleitete Acylgruppe und/oder durch Silyl geschütztes Amino, Niederalkylamino, Niederalkanoylamino oder unsubstituiertes oder durch Niederalkyl, Niederalkoxy, Hydroxy, Halogen, Nitro und/oder Amino substituiertes Phenylniederalkylamino, N-Niederalkyl-N-phenylniederalkyl-amino oder N-Niederalkanoyl-N-phenylniederalkyl-amino. dabei sind von einem Halbester der Kohlensäure abgeleitete Acylgruppen sind beispielsweise Niederalkoxycarbonyl oder unsubstituiertes oder durch Niederalkyl, Niederalkoxy, Halogen und/oder Trifluormethyl substituiertes Phenylniederalkoxycarbonyl, und Silyl insbesondere Triniederalkylsilyl, z.B. Trimethylsilyl.

Durch eine von einem Halbester der Kohlensäure abgeleitete Acylgruppe oder durch Silyl geschütztes gegebenenfalls aliphatisch oder araliphatisch substituiertes Amino ist dementsprechend beispielsweise N-Niederalkoxycarbonylamino, N-Niederalkyl-N-niederalkoxycarbonyl-amino, N-Niederalkanoyl-N-niederalkoxycarbonylamino, N-Niederalkoxycarbonyl-N-phenylniederalkylamino, N-Phenylniederalkoxycarbonylamino, N-Niederalkyl-N-phenylniederalkoxycarbonyl-amino, N-Niederalkanoyl-N-phenylniederalkoxycarbonyl-amino, N-Phenylniederalkoxycarbonyl-N-phenylniederalkylamino, N-Triniederalkylsilylamino, N-Niederalkyl-N-triniederalkylsilyl-amino, N-Niederalkanoyl-N-triniederalkylsilyl-amino oder N-Triniederalkylsilyl-N-phenylniederalkyl-amino.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Benzofuranylniederalkyl ist beispielsweise Benzofuranyl-C₁-C₄-alkyl, wie Benzofuranylmethyl 1- Benzofuranylethyl, 2- Benzofuranylethyl, 3- Benzofuranylpropyl oder 4-Benzofuranylbutyl.

Chinolinylniederalkyl ist beispielsweise Chinolinyl-C₁-C₄-alkyl, wie Chinolinylmethyl 1-Chinolinylethyl, 2- Chinolinylethyl, 3- Chinolinylpropyl oder 4-Chinolinylbutyl.

Cycloalkylniederalkyl ist beispielsweise Cyclopropyl-, Cyclobutyl-, Cyclopentyl- oder Cyclohexyl-C₁-C₄-alkyl, wie Cyclopropyl-, Cyclobutyl-, Cyclopentyl- oder Cyclohexylmethyl, 1-Cyclopropyl-, 1-Cyclobutyl-, 1-Cyclopentyl- oder 1-Cyclohexylethyl, 2-Cyclopropyl-, 2-Cyclobutyl-, 2-Cyclopentyl- oder 2-Cyclohexylethyl, 3-Cyclopropyl-, 3-Cyclobutyl-, 3-Cyclopentyl-oder 3-Cyclohexylpropyl oder 4-Cyclopropyl-, 4-Cyclobutyl-, 4-Cyclopentyl- oder 4-Cyclohexylbutyl.

Diniederalkylamino ist beispielsweise Di-C₁,-C₄-alkylamino, wie Dimethylamino, N-Methyl-N-ethylamino, Diethylamino, N-Methyl-N-propylamino oder N-Butyl-N-methyl-amino.

Furylniederalkyl ist beispielsweise Furyl-C₁-C₄-alkyl, wie Furylmethyl 1- Furylethyl, 2- Furylethyl, 3- Furylpropyl oder 4- Furylbutyl.

Halogen ist beispielsweise Halogen der Atomnummer bis und mit 35, wie Chlor oder Brom, ferner Fluor.

Indolylniederalkyl ist beispielsweise Indolyl-C₁-C₄-alkyl, wie Indolylmethyl 1-Indolylethyl, 2-Indolylethyl, 3-Indolylpropyl oder 4-Indolylbutyl

N- Niederalkyl-N-phenylniederalkoxycarbonyl-amino ist beispielsweise N-C₁-C₄-Alkyl-C₁-C₇-alkanoyl-amino, wie N-Methyl-, N-Ethyl-, N-Propyl-, N-Isopropyl-, N-Butyl- oder N-Tertiärbutyl-N-acetyl-amino oder N-Methyl-, N-Ethyl-, N-Propyl-, N-Isopropyl-, N-Butyl- oder N-Tertiärbutyl-N-pivaloyl-amino.

N-N Niederalkanoyl-N-niederalkoxycarbonyl-amino ist beispielsweise N-C₁-C₇-Alkanoyl-C₁-C₄-alkoxycarbonyl-amino, wie N-Acetyl-N-tertiärbutyloxycarbonyl-amino.

N-Niederalkanoyl-N-niederalkyl-amino ist beispielsweise N-C₁-C₇-Alkanoyl-N-C₁-C₄-alkylamino, wie N-Acetyl-N-methyl-amino, N-Acetyl-N-ethyl-amino, N-Acetyl-N-propyl-amino, N-Acetyl-N-butyl-amino, N-Pivaloyl-N-methyl-amino, N- Pivaloyl-N-ethyl-amino, N-Pivaloyl-N-propyl-amino oder N- Pivaloyl-N-butyl-amino

N-Niederalkanoyl-N-phenylniederalkoxycarbonyl-amino ist beispielsweise N-C₁-C₇-Alkanoyl-N-C₁-C₄-phenylalkoxycarbonyl-amino, wie N-Acetyl-N-benzyloxycarbonyl-amino.

N-Niederalkanoyl-N-phenylniederalkyl-amino ist beispielsweise N-C₁-C₇-Alkanoyl-N-C₁-C₄-phenylyl-amino, wie N-Acetyl-N-benzylamino oder N-Acetyl-N-(2-phenylethyl)-amino,

N-Niederalkanoyl-N-triniederalkylsilyl-amino beispielsweise N-C₁-C₇-Alkanoyl-N-C₁-C₄-tri-C₁-C₄-alkylsilyl-amino, wie N-Acetyl-N-trimethylsilyl-amino.

N-Niederalkoxycarbonyl-N-phenylniederalkylamino ist beispielsweise N-C₁-C₄-Alkoxy-carbonyl-N-phenyl-C₁-C₄-alkyl-amino, wie N-Tertiärbutyloxycarbonyl-N-benzyl-amino.

Niederalkoxy ist beispielsweise C₁-C₇-Alkoxy, vorzugsweise C₁-C₅-Alkoxy, wie Methoxy, Ethoxy, Propyloxy, Isopropyloxy, Butyloxy, Isobutyloxy, Sekundärbutyloxy, Tertiärbutyloxy, Pentyloxy oder eine Hexyloxy- oder Heptyloxygruppe.

Niederalkoxycarbonylamino ist beispielsweise C₁-C₇-Alkoxycarbonylamino, vorzugsweise C₁-C₅-Alkoxycarbonylamino, wie Methoxycarbonylamino, Ethoxycarbonylamino, Propyloxycarbonylamino, Isopropyloxycarbonylamino, Butyloxycarbonylamino, Isobutyloxycarbonylamino, Sekundärbutyloxycarbonylamino, Tertiärbutyloxyamino, Pentyloxycarbonyl amino oder eine Hexyloxycarbonylamino- oder Heptyloxycarbonylaminogruppe.

N-Niederalkyl-N-niederalkoxycarbonyl-amino ist beispielsweise N-C₁-C₄-Alkyl-N-C₁-C₄-alkoxycarbonyl-amino, wie N-Methyl-, N-Ethyl-, N-Propyl-, N-Isopropyl-, N-Butyl- oder N-Tertiärbutyl-N-tertiärbutyloxycarbonyl-amino.

N-Niederalkyl-N-phenylniederalkyl-amino ist beispielsweise N-C₁-C₄-Alkyl-N-phenyl-C₁-C₄-alkyl-amino, wie N-Methyl-, N-Ethyl-, N-Propyl-, N-Isopropyl-, N-Butyl- oder N-Tertiärbutyl-N-benzyl-amino.

N-Niederalkyl-N-triniederalkylsilyl-amino ist beispielsweise N-C₁-C₄-Alkyl-N-tri-C₁-C₄-alkylsilyl-amino, wie N-Methyl-, N-Ethyl-, N-Propyl-, N-Isopropyl-, N-Butyl- oder N-Tertiärbutyl-N-trimethylsilyll-amino.

N-Phenylniederalkoxycarbonyl-N-phenylniederalkylamino ist beispielsweise N-Phenyl-C₁-C₄-alkoxycarbonyl-N-phenyl-C₁-C₄-alkyl-amino, wie N-Benzyl-N-benzyloxycarbonyl-amino.

N-Phenylniederalkoxycarbonylamino ist beispielsweise N-Phenyl-C₁-C₄-alkoxycarbonylamino, wie N-Benzyloxycarbonylamino.

N-Triniederalkylsilyl-N-phenylniederalkylamino ist beispielsweise N-Tri-C₁-C₄-alkyl-N-phenyl-C₁-C₄-alkyl-amino, wie N-Benzyl-N-trimethylsilyl-amino.

N-Triniederalkylsilylamino ist beispielsweise N-Tri-C₁-C₄-alkylamino, wie N- Trimethylsilylamino.

Naphthylniederalkylreste sind beispielsweise Naphthyl-C₁-C₄-alkylreste, wie 1- oder 2-Naphthylmethyl, 2-(1- oder 2-Naphthyl)ethyl, 3-(1- oder 2-Naphthyl)propyl oder 4-(1- oder 2-Naphthyl)-butyl.

Niederalkanoylamino ist beispielsweise N-C₁-C₇-Alkanoylamino, wie Acetylamino oder Pivaloylamino.

Niederalkenyl ist beispielsweise C₁-C₇-Alkenyl, wie Vinyl oder Allyl.

Niederalkoxy ist beispielsweise C₁-C₇-Alkoxy, vorzugsweise C₁-C₅-Alkoxy, wie Methoxy, Ethoxy, Propyloxy, Isopropyloxy, Butyloxy, Isobutyloxy, Sekundärbutyloxy, Tertiärbutyloxy, Pentyloxy oder eine Hexyloxy- oder Heptyloxygruppe.

Niederalkyl kann geradkettig oder verzweigt und/oder überbrückt sein und ist beispielsweise entsprechendes C₁-C₇-Alkyl, wie Methyl, Ethyl, Propyl, Isopropyl, Butyl, Isobutyl, Sekundärbutyl, Tertiärbutyl, oder eine Pentyl-, Hexyl- oder Heptylgruppe. Niederalkyl R₂ bzw. R₃ ist insbesondere C₂-C₇-Alkyl, Niederalkyl R₅ bzw. R₇ ist insbesondere verzweigtes C₃-C₇-Alkyl und Niederalkyl R₈ bzw. R₃ ist beispielsweise geradkettiges, verzweigtes oder überbrücktes C₃-C₇-Alkyl.

Niederalkylamino ist beispielsweise C₁-C₄-Alkylamino, wie Methylamino, Ethylamino, Propylamino, Butylamino, Isobutylamino, Sekundärbutylamino oder Tertiärbutylamino.

Niederalkylthio ist beispielsweise C₁-C₄-Alkylthio, wie Methylthio, Ethylthio, Propylthio, Butylthio, Isobutylthio, Sekundärbutylthio oder Tertiärbutylthio.

Phenylniederalkyl ist beispielsweise Phenyl-C₁-C₄-alkyl, wie Benzyl, 1- oder 2-Phenethy, 3-Phenylpropylamino oder 4-Phenylbutyl.

Phenylniederalkylamino ist beispielsweise Phenyl-C₁-C₄-alkylamino, wie Benzylamino, 1- oder 2-Phenethylamino, 3-Phenylpropylamino oder 4-Phenylbutylamino.

Pyridylniederalkyl ist beispielsweise Pyridinyl-C₁-C₄-alkyl, wie Pyridinylmethyl, 1- oder 2-Pyridinylethyl, 3-Pyridinylpropyl oder 4-Pyridinylbutyl.

Pyrimidinylniederalkyl ist beispielsweise Pyrimidinyl-C₁-C₄-alkyl, wie Pyrimidinylmethyl, 1- oder 2-Pyrimidinylethyl, 3-Pyrimidinylpropyl oder 4-Pyrimidinylbutyl.

Thienylniederalkyl ist beispielsweise Thienyl-C₁-C₄-alkyl, wie Thienylmethyl, 1- oder 2-Thienylethyl, 3-Thienylpropyl oder 4-Thienylbutyl.

Salze von Verbindungen mit salzbildenden Gruppen sind insbesondere Säureadditionssalze, Salze mit Basen oder bei Vorliegen mehrerer salzbildender Gruppen gegebenenfalls auch Mischsalze oder innere Salze.

Solche Salze werden beispielsweise von Verbindungen der Formel I mit einer sauren Gruppe, z.B. einer Carboxygruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, z.B. Alkalimetall-, insbesondere Lithium-, Natrium- oder Kaliumsalze, Erdalkalimetallsalze, beispielsweise Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder Triniederalkylaminen, oder mit quaternären Ammoniumbasen gebildet werden, z.B. Methyl-, Ethyl-, Diethyl- oder Triethylamin, Mono-, Bis- oder Tris-(2-hydroxyniederalkyl)-aminen, wie Ethanol-, Diethanol- oder Triethanolamin, Tris-(hydroxymethyl)-methylamin oder 2-Hydroxytert-butylamin, N,N-Diniederalkyl-N-(hydroxyniederalkyl)-amin, wie N,N-DiN-Dimethyl-N-(2-hydroxyethyl)-amin, oder N-Methyl-D-glucamin, oder quaternären Ammoniumhydroxiden, wie Tetrabutylammoniumhydroxid.

Die Verbindungen der Formel I mit einer basischen Gruppe, z.B. einer Aminogruppe, können Säureadditionssalze bilden, z.B. mit geeigneten anorganischen Säuren, z.B. Halogenwasserstoffsäure, wie Salzsäure, Bromwasserstoffsäure, Schwefelsäure unter Ersatz eines oder beider Protonen, Phosphorsäure unter Ersatz eines oder mehrerer Protonen, z.B. Orthophosphorsäure, Metaphosphorsäure oder Pyrophosphorsäure, unter Ersatz eines oder mehrerer Protonen, oder mit organischen Carbon-, Sulfon-, Sulfo- oder Phosphonsäuren oder N-substituierter Sulfaminsäuren, z.B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure, Isonicotinsäure, ferner Aminosäuren, wie natürlich vorkommenden α-Aminosäuren, sowie Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3- Phosphoglycerat, Glucose-6-Phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung und Reinigung können auch pharmazeutisch ungeeignete Salze Verwendung finden.

Die Verbindungen der Formel I sind wertvolle Zwischenprodukte für die Herstellung von Arzneimittelwirkstoffen, beispielsweise von Verbindungen der Formeln IIa und IIb 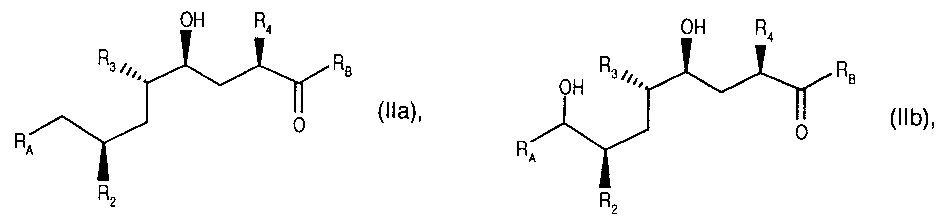
worin
R_{A} einen aromatischen oder heteroaromatischen Rest bedeutet,
R₂ und R₄ unabhängig voneinander aliphatische, cycloaliphatische, cycloaliphatisch-aliphatische oder araliphatische Reste darstellen,
R₃ für unsubstituiertes oder N-mono- oder N,N-diniederalkyliertes oder N-niederalkanoyliertes Amino steht und
R_{B} eine aliphatisch, cycloaliphatisch oder heteroaromatisch-aliphatisch substituierte Aminogruppe bedeutet,
und ihrer Salze, die beispielsweise als Antihypertensiva eingesetzt werden können.

Die Verbindungen der Formel I sind insbesondere wertvolle Zwischenprodukte für die Herstellung von δ-Amino-γ-hydroxy-ω-aryl-alkansäureamiden der Formel IIc 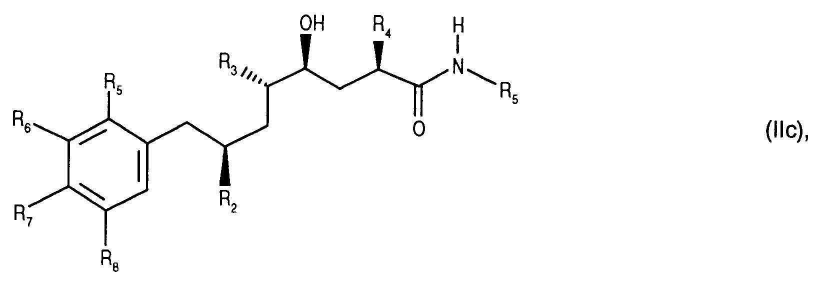
worin
R₅ Wasserstoff, Hydroxy, Niederalkoxy, Cycloalkoxy, Niederalkoxyniederalkoxy oder gegebenenfalls verestertes oder amidiertes Carboxyniederalkoxy bedeutet,
R₆ Wasserstoff, Niederalkyl, Cycloalkyl, Niederalkoxyniederalkyl, Niederalkoxyniederalkoxy niederalkyl, Cycloalkoxyniederalkyl, Hydroxy, gegebenenfalls niederalkanoyliertes, halogeniertes oder sulfonyliertes Hydroxyniederalkoxy, gegebenenfalls durch Niederalkyl, Niederalkanoyl und/oder Niederalkoxycarbonyl substituiertes Aminoniederalkyl, gegebenenfalls hydriertes Heteroarylniederalkyl, Niederalkoxyiminoniederalkyl, durch Nieder alkyl, Niederalkanoyl und/oder Niederalkoxycarbonyl substituiertes Aminoniederalkoxy, Oxoniederalkoxy, Niederalkoxy, Cycloalkoxy, Niederalkenyloxy, Cycloalkoxyniederalkoxy, Niederalkoxyniederalkoxy, Niederalkoxyniederalkenyl, Niederalkenyloxyniederalkoxy, Niederalkoxyniederalkenyloxy, Niederalkenyloxyniederalkyl, Niederalkanoylniederalkoxy, gegebenenfalls S-oxidiertes Niederalkylthioniederalkoxy, Niederalkyl thio(hydroxy)niederalkoxy, Arylniederalkoxy, gegebenenfalls hydriertes Heteroarylniederalkoxy, Cyanoniederalkoxy, gegebenenfalls verestertes oder amidiertes Carboxyniederalkoxy oder gegebenenfalls verestertes oder amidiertes Carboxyniederalkyl bedeutet,
R₇ gegebenenfalls halogeniertes Niederalkyl, Niederalkoxyniederalkyl, Cycloalkoxyniederalkyl, Hydroxyniederalkyl, gegebenenfalls S-oxidiertes Niederalkylthioniederalkyl, gegebenenfalls hydriertes Heteroarylthioniederalkyl, gegebenenfalls hydriertes Heteroarylniederalkyl, gegebenenfalls N-mono- oder N,N-diniederalkyliertes, N-niederalkanoyliertes, N-niederalkansulfonyliertes oder durch Niederalkylen, gegebenenfalls N'-niederalkyliertes oder N'-niederalkanoyliertes Azaniederalkylen, Oxaniederalkylen oder gegebenenfalls S-oxidiertes Thianiederalkylen Thianiederalkylen N,N-disubstituiertes Aminoniederalkyl, Cyanoniederalkyl, gegebenenfalls verestertes oder amidiertes Carboxyniederalkyl, Cycloalkyl, Aryl, Hydroxy, Niederalkoxy, Cycloalkoxy, Niederalkoxyniederalkoxy, Cycloalkoxyniederalkoxy, Hydroxyniederalkoxy, Arylniederalkoxy, gegebenenfalls halogeniertes Niederalkoxy, gegebenenfalls S-oxidiertes Niederalkylthioniederalkoxy, gegebenenfalls hydriertes Heteroarylniederalkoxy, gegebenenfalls hydriertes Heteroarylthioniederalkoxy, gegebenenfalls N-mono- oder N,N-diniederalkyliertes, N-niederalkanoyliertes, N-niederalkansulfonyliertes oder durch Niederalkylen, gegebenenfalls N'-niederalkyliertes oder N'-niederalkanoyliertes Azaniederalkylen, Oxaniederalkylen oder gegebenenfalls S-oxidiertes Thianiederalkylen substituiertes Aminoniederalkoxy, Cyanoniederalkoxy oder gegebenenfalls verestertes oder amidiertes Carboxyniederalkoxy bedeutet oder gemeinsam mit R₈ Niederalkylen dioxy oder einen ankondensierten Benzo- oder Cyclohexenoring darstellt,
R₈ gemeinsam mit R₇ Niederalkylendioxy oder einen ankondensierten Benzo- oder Cyclohexenoring darstellt oder für Wasserstoff, Niederalkyl, Hydroxy, Niederalkoxy, Cycloalkoxy steht,
R₂ für Niederalkyl oder Cycloalkyl steht,
R₆ gegebenenfalls N-mono- oder N,N-diniederalkyliertes oder N-niederalkanoyliertes Amino bedeutet,
R₄ Niederalkyl, Niederalkenyl, Cycloalkyl oder Arylniederalkyl bedeutet und
R₆ Niederalkyl, Cycloalkyl, gegebenenfalls aliphatisch verestertes oder verethertes Hydroxyniederalkyl, gegebenenfalls N-niederalkanoyliertes, N-mono- oder N,N-diniederalkyliertes oder durch Niederalkylen, Hydroxy-, Niederalkoxy- oder Niederalkanoyloxyniederalkylen, gegebenenfalls N'-niederalkanoyliertes bzw. N'-niederalkyliertes Azaniederalkylen, Oxaniederalkylen oder gegebenenfalls S-oxidiertes Thianiederalkylen N,N-disubstituiertes Aminoniederalkyl, gegebenenfalls verestertes oder amidiertes Carboxyniederalkyl, gegebenenfalls verestertes oder amidiertes Dicarboxyniederalkyl, gegebenenfalls verestertes oder amidiertes Carboxy(hydroxy)niederalkyl, gegebenenfalls verestertes oder amidiertes Carboxycyloalkylniederalkyl, Cyanoniederalkyl, Niederalkansulfonylniederalkyl, gegebenenfalls N-mono- oder N,N-diniederalkyliertes Thiocarbamoylniederalkyl, gegebenenfalls N-mono- oder N,N-diniederalkyliertes Sulfamoylniederalkyl oder einen über ein C-Atom gebundenen und gegebenenfalls hydrierten und/oder oxosubstituerten Heteroarylrest oder einen durch einen über ein C-Atom gebundenen und gegebenenfalls hydrierten und/oder oxosubstituerten Heteroarylrest substituiertes Niederalkyl darstellt, und ihre Salze, die als Reninhemmer wirken und deshalb als Antihypertensiva eingesetzt werden können.

Die Verbindungen der Formel I sind in allererster Linie geeignet für die Herstellung von Verbindungen der Formel IIa, worin
R₅ und R₈ Wasserstoff bedeuten,
R₆ für C₁-C₄-Alkoxy-C₁-C₄-alkoxy, wie 3-Methoxypropyloxy, oder C₁-C₄-Alkoxy-C₁-C₄-alkyl, wie 3-Methoxybutyl, steht,
R₇ C₁-C₄-Alkyl, wie Isopropyl oder Tertiärbutyl, oder C₁-C₄-Alkoxy, wie Methoxy, darstellt,
R₃ Amino ist,
R₂ und R₄ verzweigtes C₁-C₄-Alkyl, wie Isopropyl, bedeuten und R₆ Carbamoyl-C₁-C₄-alkyl, wie 2- oder 3-Carbamoylpropyl, 2-(3-Carbamoyl)propyl oder 1-(2-Carbamoyl-2-methyl)propyl, N-C₁-C₄-Alkylcarbamoyl-C₁-C₄-alkyl, wie 3-(N-Methylcarbamoyl)propyl, 2-[1-(N-Methylcarbamoyl)]propyl, 1-[2-(N-Methylcarbamoyl)] propyl, vor allem 1-[2(R)-(N-Methylcarbamoyl)]propyl, N,N-Di-C₁-C₄-alkylcarbamoyl-C₁-C₄-alkyl, wie N,N-Dimethyl carbamoylmethyl oder 2-(N,N-Dimethylcarbamoyl)ethyl, 3-(N,N-Dimethylcarbamoyl) propyl, Morpholino-C₁-C₄-alkyl, wie 2-Morpholinoethyl, 3-Morpholinopropyl oder 1-(2-Morpholino-2-methyl)propyl, Thiomorpholino-C₁-C₄-alkyl, wie 2-Thiomorpholinoethyl, 4-(1-C₁-C₄-Alkanoylpiperidyl)-C₁-C₄-alkyl, wie 2-[4-(1-Acetyl)piperidinyl]ethyl, 2-Oxopyrrolidinyl-C₁-C₄-alkyl, wie 2-Oxopyrroli din-5(S)-ylmethyl oder 2-Oxopyrrolidin-5(R)-ylmethyl, bedeutet,
und ihrer Salze.

Die Überführung von Zwischenprodukten der Formel I, insbesondere von solchen. worin R₃ Azido darstellt, in Reninhemmer der Formel II erfolgt in üblicher Weise, beispielsweise indem man eine entsprechende Verbindung der Formel I, worin
R₁ für gegebenenfalls verestertes Carboxy steht,
in üblicher Weise, insbesondere durch Überführung in das entsprechende Säurechlorid, beispielsweise durch Umsetzung mit Oxalylchlorid, und anschließende Reduktion z.B. mittels Lithiumtritertiärbutyloxyaluminiumhydrid in Tetrahydrofuran, oder eine Verbindung der Formel I, worin R₁ Hydroxymethyl bedeutet, durch Oxidation, z.B. mittels Pyridin/Schwefeltrioxid in Dimethylsulfoxid/Dichlormethan, in den entsprechenden Aldehyd der Formel la 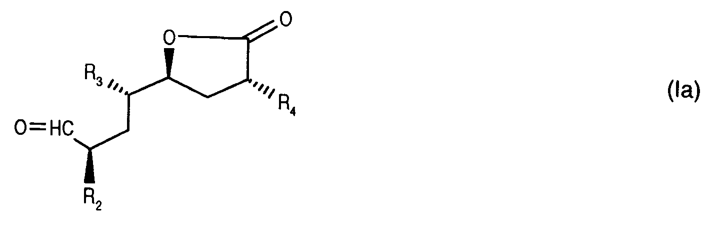
überführt, diesen in üblicher Weise, beispielsweise in Tetrahydrofuran unter Kühlung, z.B. auf -70°C bis -80°C, mit einer Halogenmagnesiumverbindung der Formel R_{A}-Mg-Hal (Ic) kondensiert, die erhaltene Verbindungen der Formel Id 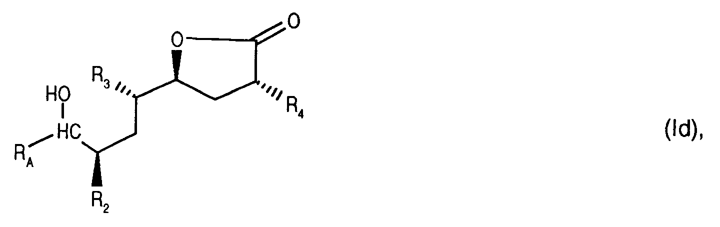
beispielsweise durch Umsetzung mit Acetanhydrid oder Isobutteräureanhydrid, insbesondere in Gegenwart von Dimethylaminopyridin in Dichlormethan, an der Hydroxygruppe intermediär schützt, die erhaltene Verbindung der Formel le 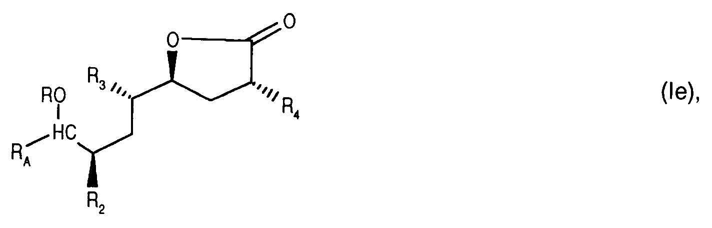
worin R eine Hydroxyschutzgruppe, z.B. Acetyl oder Isobutyryl, bedeutet, mit einem Amin der Formel H-R_{E} (If) umsetzt und die erhaltene Verbindung der Formel Ig 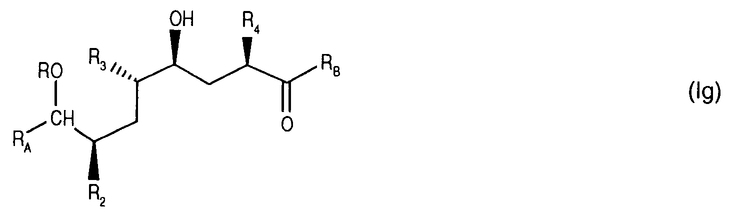
katalytisch, beispielsweise in Gegenwart von Palladium oder bevorzugt Platinoxid auf Kohle bei Raumtemperatur und Normaldruck, hydriert, wobei gegebenenfalls die Gruppe R-O- durch Wasserstoff ersetzt sowie eine gegebenenfalls vorhandene Azidogruppe R₃ zu Amino reduziert und die entsprechende Verbindung der Formel IIa, IIb bzw. IIc, worin R₃ für unsubstituiertes Amino steht erhalten wird, die dann gewünschtenfalls N-mono- oder N,N-diniederalkyliert oder N-niederalkanoyliert werden kann.

Die Erfindung betrifft in erster Linie Verbindungen der Formel I, worin
R₁ Carboxy, Niederalkoxycarbonyl, unsubstituiertes oder durch Niederalkyl, Niederalkoxy, Halogen und/oder Trifluormethyl substituiertes Phenylniederalkyoxycarbonyl, Hydroxymethyl oder Formyl darstellt,
R₂ und R₄ unabhängig voneinander Niederalkylreste, Niederalkenyl, Niederalkoxy oder Niederalkylthio, 3- bis 7-gliedrige Cycloalkylniederalkylreste, unsubstituierte oder durch Niederalkyl, Niederalkoxy, Hydroxy, Halogen, Nitro und/oder Amino substituierte Phenylniederalkyl- oder Naphthylniederalkylreste oder unsubstituiertes oder durch Niederalkyl, Niederalkoxy, Hydroxy, Halogen, Nitro und/oder Amino substituiertes Pyridylniederalkyl, Indolylniederalkyl, Chinolinylniederalkyl, Pyrimidinylniederalkyl, Furylniederalkyl, Benzofuranylniederalkyl oder Thienylniederalkyl bedeuten und
R₃ Azido, Niederalkylamino, Diniederalkylamino, Niederalkanoylamino oder N-Niederalkanoyl-N-niederalkyl-amino, unsubstituiertes oder durch Niederalkyl, Niederalkoxy, Hydroxy, Halogen, Nitro und/oder Amino substituiertes Phenylniederalkylamino, N-Niederalkyl-N-phenylniederalkyl-amino oder N-Niederalkanoyl-N-phenylniederalkyl-amino oder N-Niederalkoxycarbonylamino, N-Niederalkyl-N-niederalkoxycarbonyl-amino, N-Niederalkanoyl-N-niederalkoxycarbonyl-amino, N-Niederalkoxycarbonyl-N-phenylniederalkylamino, N-Phenylniederalkoxycarbonylamino, N- Niederalkyl-N-phenylniederalkoxycarbonyl-amino, N-Niederalkanoyl-N-phenylniederalkoxycarbonyl-amino, N- Phenylniederalkoxycarbonyl-N-phenylniederalkylamino, N-Triniederalkylsilylamino, N-Niederalkyl-N-triniederalkylsilyl-amino, N-Niederalkanoyl-N-triniederalkylsilyl-amino oder N-Triniederalkylsilyl-N-phenylniederalkylamino darstellt,
und ihre Salze.

Die Erfindung betrifft in erster Linie beispielsweise Verbindungen der Formel I, worin
R₁ Carboxy, Niederalkoxycarbonyl, Hydroxymethyl oder Formyl darstellt,
R₂ für Niederalkyl steht,
R₃ Azido darstellt und
R₄ Niederalkyl oder unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenyl- oder Naphthylniederalkyl bedeutet,
und und ihre Salze.

Die Erfindung betrifft vor allem Verbindungen der Formel I, worin
R₁ Carboxy bedeutet,
R₂ und R₄ C₁-C₄-Alkyl, vorzugsweise Methyl oder verzweigtes C₁-C₄-Alkyl, wie Isopropyl, C₂-C₄-Alkenyl, wie Allyl oder Methallyl, C₁-C₅-Alkoxy, wie Methoxy, Ethoxy, Propyloxy, Isopropyloxy, Butyloxy, Isobutyloxy, Sekundärbutyloxy, Tertiärbutyloxy oder Pentyloxy, C₁-C₅-Alkylthio, wie Methylthio, Ethylthio, Propylthio, Isopropylthio, Butylthio, Isobutylthio, Sekundärbutylthio, Tertiärbutylthio oder Pentylthio, 3- bis 7-gliedrige Cycloalkyl-C₁-C₄-alkyl, wie Cyclopropyl- Cyclobutyl-, Cyclopentyl- oder Cyclohexylmethyl, unsubstituierte oder durch C₁-C₄-Alkyl, wie Methyl, C₁-C₄-Alkoxy, wie Methoxy, Hydroxy, Halogen der Atomnummer bis und mit 35, wie Chlor oder Brom, Nitro und/oder Amino substituierte Phenyl-C₁-C₄-alkyl bedeutet und
R₃ Azido, C₁-C₄-Alkoxycarbonylamino oder Phenyl- C₁-C₄-Ikoxycarbonylamino ist,
und und ihre Salze.

Die Erfindung betrifft in allererster Linie Verbindungen der Formel I, worin
R₁ Carboxy bedeutet,
R₂ C₁-C₄-Alkyl, vorzugsweise Methyl oder verzweigtes C₁-C₄-Alkyl, wie Isopropyl, bedeutet,
R₃ Azido ist und
R₄ C₁-C₄-Alkyl, vorzugsweise Methyl oder verzweigtes C₁-C₄-Alkyl, wie Isopropyl, C₂-C₄-Alkenyl, wie Allyl oder Methallyl, oder unsubstituiertes oder durch C₁-C₄-Alkyl, wie Methyl, C₁-C₄-Alkoxy, wie Methoxy, Hydroxy, Halogen der Atomnummer bis und mit 35, wie Chlor oder Brom, Nitro und/oder Amino substituiertes Phenyl-C₁-C₄-alkyl bedeutet, und ihre Salze.

Die Erfindung betrifft namentlich die in den Beispielen genannten Verbindungen der Formel I und ihre Salze.

Das erfindungsgemäße Verfahren zur Herstellung von Verbindungen der Formel I ist dadurch gekennzeichnet, daß man in einer Verbindung der Formel III 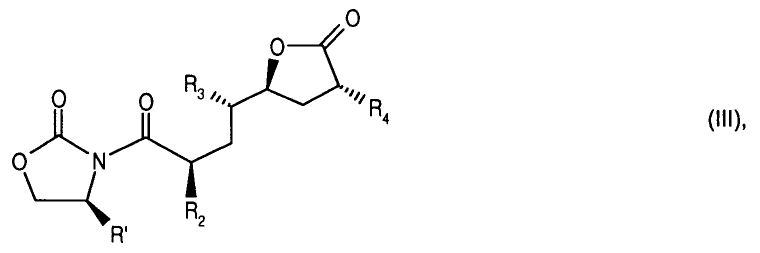
worin
R' einen gegebenenfalls substituierten Kohlenwasserstoffrest und
R₃ beispielsweise Azido bedeutet, wobei
R₂ und R₄ die angegebenen Bedeutungen haben,
die 4-R'-2-oxo-oxazolidin-1-ylcarbonylgruppe selektiv zu Carboxy hydrolysiert, einen dabei gegebenenfalls geöffneten Laktonring unter Verwendung eines sauren Katalysators wieder schließt und gewünschtenfalls eine erfindungsgemäße Verbindung der Formel I in eine andere erfindungsgemäße Verbindung der Formel I umwandelt, ein gegebenenfalls erhältliches Isomerengemisch auftrennt und/oder die verfahrensgemäß erhältliche Verbindung der Formel I in ein Salz oder ein verfahrensgemäß erhältliches Salz in die freie Verbindung oder in ein anderes Salz überführt.

Als Kohlenwassertoffreste kommen beliebige aliphatische, cycloaliphatische, cycloaliphatisch-aliphatische, araliphatische oder aromatische Kohlenwassertoffreste, wie Niederalkyl, Niederalkenyl, 3- bis 5-gliedriges Cycloalkyl, 3- bis 5-gliedriges Cycloalkylniederalkyl, Phenylniederalkyl, wie insbesondere Benzyl, Naphthylniederalkyl, Phenyl oder Naphthyl in Betracht. Substituenten, insbesondere von Phenylniederalkyl, Naphthylniederalkyl, Phenyl oder Naphthyl sind beispielsweise Niederalkyl, Niederalkoxy, Halogen, Trifluormethyl, Nitro, Amino oder Mono- oder Diniederalkylamino.

Die Hydrolyse der 4-R'-2-oxo-oxazolidin-1 -ylcarbonylgruppe erfolgt beispielsweise durch Behandeln mit einem Alkalimetallhydroxid in Gegenwart eines basischen Hydrolysemittels, insbesondere von Lithiumhydroxid in Gegenwart von Wasserstoffperoxid.
Wasserstoffperoxid wird beispielsweise im Überschuß einer etwa 10 Vol.-%-iger, beispielsweise 7.5 Vol.-%-iger bis 12.5 Vol.-%-iger, wäßrigen Lösung verwendet.

Die Umsetzung erfolgt in üblicher Weise, beispielsweise im Temperaturbereich von etwa - 25 ° C bis etwa +10 ° C, insbesondere von etwa -5 ° C bis etwa +5 ° C, vorteilhaft in Gegenwart eines gegenüber Wasserstoffperoxid inerten, mit Wasser mischbaren organischen Lösungsmittels, wie eines cyclischen Ethers, insbesondere von Tetrahydrofuran oder Dioxan. Nach Zersetzung des überschüssigen Wasserstoffperoxides, beispielsweise mittels Natriumbisulfit, erfolgt die Aufarbeitung in üblicher Weise.

Ausgangsstoffe der Formel III werden beispielsweise hergestellt, indem man ein 1,4-Dihalogenbuten, z.B. 1,4-Dibrombuten, zunächst mit einer Verbindung der Formel IVa und anschließend mit einer Verbindung der Formel IVb bzw. zunächst mit einer Verbindung der Formel IVb und anschließend mit einer Verbindung der Formel IVa 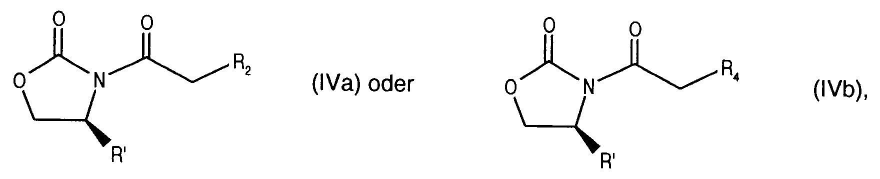
oder in einem Schritt oder schrittweise mit der etwa doppeltmolaren Menge einer Verbindung der Formel IVa oder IVb umsetzt, worin R', R₂ und R₄ die angegebenen Bedeutungen haben und die erhaltene Verbindung der Formel V 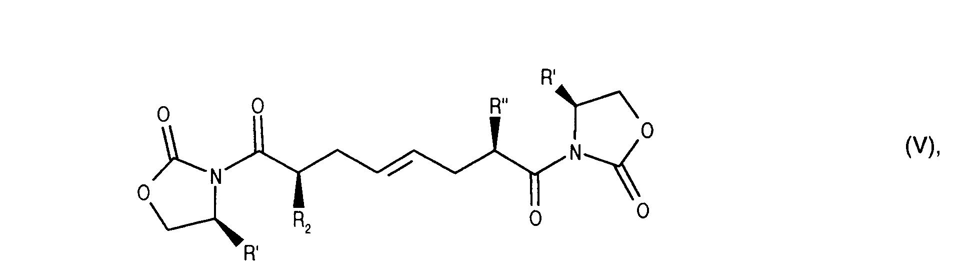
worin R'' für Reste R₂ oder R₄ steht, beispielsweise durch Behandeln mit einem üblichen Halogenierungsmittel, wie elementarem Halogen, insbesondere Brom oder Jod, oder vorzugsweise mit einem N-Halogensuccinimid, insbesondere N-Bromsuccinimid in 1,2-Dimethoxyethan in die entsprechende Verbindung der Formel VI 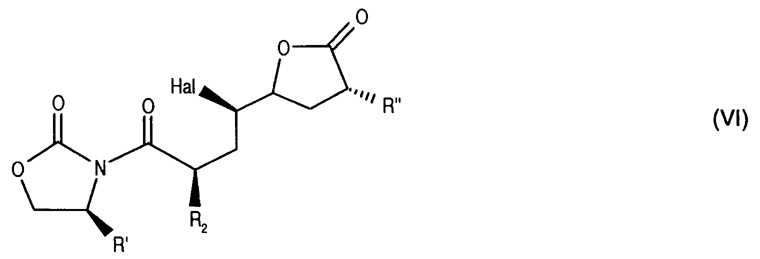
worin Hal Halogen, wie Brom, bedeutet, überführt, das gewünschte Isomere, in dem R'' für R₄ steht, abtrennt, in diesem das Halogenatom, beispielsweise durch Behandeln mit Tetrabenzylammoniumazid in Toluol, unter Inversion durch Azido ersetzt und gewünschtenfalls die Azidogruppe zu Amino hydriert und anschließend aliphatisch oder araliphatisch substituiert und/oder durch eine Aminoschutzgruppe schützt.

In einer bevorzugten Ausführungsform zur Herstellung von Verbindungen der Formel I, worin R₃ für Azido steht, wird E-1,4-Dibrombut-2-en mit der mindestens doppeltmolaren Menge einer Verbindung der Formel IVa direkt zur entsprechenden Verbindung der Formel VI, worin R'' eine Gruppe R₄ ist, umgesetzt. Dabei verläuft die Umsetzung von E-1,4-Dibrombut-2-en mit Verbindungen der Formeln IVa sowie die Weiterumsetzung der Verbindung der Formel V und der Austausch von Halogen gegen Azido weitgehend stereoselektiv.

Verfahrensgemäß erhältliche Verbindungen der Formel I können gewünschtenfalls in andere Verbindungen der Formel I umgewandelt werden.

So kann man in Verbindungen der Formel I, worin R₁ verestertes Carboxy ist, die veresterte Carboxygruppe in üblicher Weise, beispielsweise durch basische oder saure Hydrolyse, in Carboxy überführen. Umgekehrt kann man Carboxy R₁ in üblicher Weise, beispielsweise durch Umsetzung mit einem Alkohol in Gegenwart eines sauren Mittels, oder durch Überführung in Chlorcarbonyl und Weiterumsetzung mit einem Alkohol in Gegenwart eines basischen Kondensationsmittels verestern. Gewünschtenfalls kann man ein Zwischenprodukt der Formel III unter Abspaltung der 4-R'-2.oxo-oxazolidingruppe in Gegenwart geeigneter Katalysatoren, wie Titantetraisopropanolat, direkt in Verbindungen der Formel I umwandeln, worin R₁ verestertes Carboxy ist.

Auch kann man Verbindungen der Formel I, worin R₁ verestertes Carboxy ist, in üblicher Weise, beispielsweise mittels Dibutylaluminiumhydrid in Toluol oder durch Umsetzung mit Oxalylchlorid, und anschließende Reduktion z.B. mittels Natriumtritertiärbutyloxyaluminiumhydrid in Tetrahydrofuran zum entsprechenden Aldehyd, worin R₁ Formyl ist, reduziert werden. Verbindungen der Formel I, worin R₁ Carboxy ist, können durch Umsetzung mit einem Chlorameisensäureester und anschließende Reduktion des gebildeten gemischten Anhydrides, z.B. mit Natriumboranat, ebenfalls zu Aldehyden reduziert werden.

Weiterhin kann man in erhaltenen Verbindungen der Formel I, worin R₃ Azido ist, die Azidogruppe katalytisch, beispielsweise in Gegenwart von Palladium auf Kohle bei Raumtemperatur und Normaldruck, zu Amino reduzieren und die Aminogruppe N-mono- oder N,N-diniederalkylieren bzw. N-niederalkanoylieren.

Ferner kann man verfahrensgemäß erhältliche Salze von Verbindungen der Formeln I in an sich bekannter Weise in die freien Verbindungen umwandeln, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formeln I, einschließlich ihrer Salze, können auch in Form von Hydraten erhalten werden oder das zur Kristallisation verwendete Lösungsmittel einschließen.

Infolge der engen Beziehung zwischen den neuen Verbindungen in freier Form und in Form ihrer Salze sind vorstehend und nachfolgend unter den freien Verbindungen und ihren Salzen sinn- und zweckgemäß gegebenenfalls auch die entsprechenden Salze bzw. freien Verbindungen zu verstehen.

Die Erfindung betrifft auch diejenigen Ausführungsformen des Verfahrens, bei denen man von einer auf irgend einer Stufe als Zwischenprodukt erhältlichen Verbindung ausgeht und die fehlenden Schritte durchführt oder das Verfahren auf irgendeiner Stufe abbricht oder einen Ausgangsstoff unter den Reaktionsbedingungen bildet oder in Form eines reaktionsfähigen Derivates oder Salzes verwendet oder eine nach dem erfindungsgemässen Verfahren erhältliche Verbindung unter den Verfahrensbedingungen erzeugt und in situ weiterverarbeitet. Dabei geht man vorzugsweise von solchen Ausgangsstoffen aus, die zu den oben als sehr bevorzugt oder ganz besonders bevorzugt beschriebenen Verbindungen führen.

Die neuen Ausgangsstoffe, die speziell für die Herstellung der erfindungsgemäßen Verbindungen entwickelt wurden, insbesondere die zu den eingangs als bevorzugt gekennzeichneten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Dies betrifft insbesondere Verbindungen der Formeln III, V und VI, die sich, wie erwähnt, als Zwischenprodukte zur Herstellung von Verbindungen der Formel I eignen.

Die Erfindung betrifft dementsprechend auch Verbindungen Formeln III 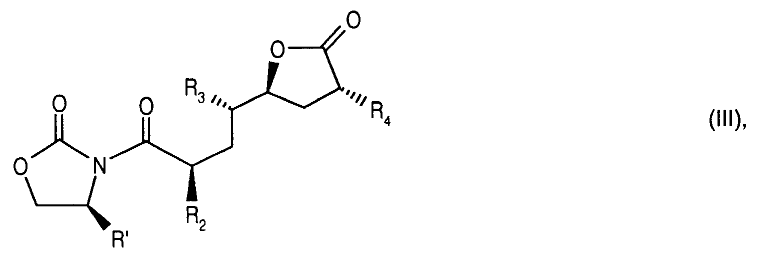 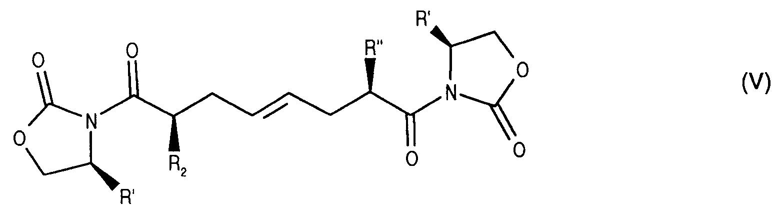
und 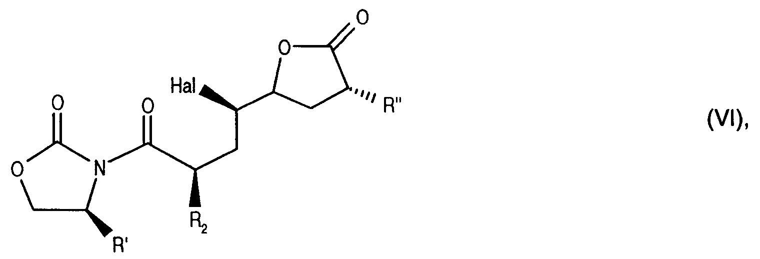
worin R₂, R₄ und R'' unabhängig voneinander aliphatische, cycloaliphatisch-aliphatische, araliphatische oder heteroarylaliphatische Reste bedeuten,
R₃ Azido oder aliphatisch oder araliphatisch substituiertes und/oder durch eine Aminoschutzgruppe geschütztes Amino darstellt,
R' einen aliphatischen, cycloaliphatischen oder cycloaliphatisch-aliphatischen Kohlenwassertoffrest oder einen unsubstituierten oder durch Niederalkyl, Niederalkoxy, Halogen, Trifluormethyl, Nitro, Amino und/oder Mono- oder Diniederalkylamino substituierten araliphatische oder aromatische Kohlenwassertoffrest bedeutet und
Hal Halogen darstellt,
und ihre Salze, Verfahren zur Herstellung derselben und ihre Verwendung als Zwischenprodukte für die Herstellung von Arzneimittelwirkstoffen.

In den erfindungsgemäss bereitgestellten Verbindungen der Formeln III, V und VI hat die Variable R₂ vorzugsweise die unter der Formel I, die Variable R' vorzugsweise die unter der Formel III angegebene Bedeutung, die Variable R'' eine der unter der Formel I für R₂ bzw. R₄ angegebenen Bedeutungen und ist Hal vorzugsweise Halogen der Atomnummer bis und mit 35.

Die Erfindung betrifft in erster Linie Verbindungen der Formeln III, V und VI, worin R₂, R₄ und R'' unabhängig voneinander Niederalkyl, Niederalkenyl, Niederalkoxy oder Niederalkylthio, 3- bis 7-gliedrige Cycloalkylniederalkylreste, unsubstituierte oder durch Niederalkyl, Niederalkoxy, Hydroxy, Halogen, Nitro und/oder Amino substituierte Phenylniederalkyl- oder Naphthylniederalkylreste oder unsubstituiertes oder durch Niederalkyl, Niederalkoxy, Hydroxy, Halogen, Nitro und/oder Amino substituiertes Pyridylniederalkyl, Indolylniederalkyl, Chinolinylniederalkyl, Pyrimidinylniederalkyl, Furylniederalkyl, Benzofuranylniederalkyl oder Thienylniederalkyl bedeuten,
R₃ Azido, Niederalkylamino, Diniederalkylamino, Niederalkanoylamino oder N-Niederalkanoyl-N-niederalkyl-amino, unsubstituiertes oder durch Niederalkyl, Niederalkoxy, Hydroxy, Halogen, Nitro und/oder Amino substituiertes Phenylniederalkylamino, N-Niederalkyl-N-phenylniederalkyl-amino oder N-Niederalkanoyl-N-phenylniederalkyl-amino oder N-Niederalkoxycarbonylamino, N-Niederalkyl-N-niederalkoxycarbonyl-amino, N-Nieder-alkanoyl-N-niederalkoxycarbonyl-amino, N-Niederalkoxycarbonyl-N-phenylniederalkylamino, N-Phenylniederalkoxycarbonylamino, N- Niederalkyl-N-phenylniederalkoxycarbonyl-amino, N-Niederalkanoyl-N-phenylniederalkoxycarbonyl-amino, N-Phenylniederalkoxycarbonyl-N-phenylniederalkylamino, N-Triniederalkylsilylamino, N-Niederalkyl-N-triniederalkylsilyl-amino, N-Niederalkanoyl-N-triniederalkylsilyl-amino oder N-Triniederalkylsilyl-N-phenylniederalkylamino darstellt und
R'' Niederalkyl, Niederalkenyl, 3- bis 5-gliedriges Cycloalkyl, 3- bis 5-gliedriges Cycloalkylniederalkyl, unsubstituiertes oder im Phenylteil durch Niederalkyl, Niederalkoxy, Halogen, Trifluormethyl, Nitro, Amino und/oder Mono- oder Diniederalkylamino substituiertes Phenylniederalkyl, unsubstituiertes oder im Naphthylteil durch Niederalkyl, Niederalkoxy, Halogen, Trifluormethyl, Nitro, Amino und/oder Mono- oder Diniederalkylamino substituiertes Naphthylniederalkyl oder unsubstituiertes oder durch Niederalkyl, Niederalkoxy, Halogen, Trifluormethyl, Nitro, Amino und/oder Mono- oder Diniederalkylamino substituiertes, Phenyl oder Naphthyl bedeutet,
und ihre Salze.

Die Erfindung betrifft vor allem Verbindungen der Formel III, V und VI, worin
R₂,
R₄ und R'' C₁-C₄-Alkyl, vorzugsweise Methyl oder verzweigtes C₁-C₄-Alkyl, wie Isopropyl, C₂-C₄-Alkenyl, wie Allyl oder Methallyl, C₁-C₅-Alkoxy, wie Methoxy, Ethoxy, Propyloxy, Isopropyloxy, Butyloxy, Isobutyloxy, Sekundärbutyloxy, Tertiärbutyloxy oder Pentyloxy, C₁-C₅-Alkylthio, wie Methylthio, Ethylthio, Propylthio, Isopropylthio, Butylthio, Isobutylthio, Sekundärbutylthio, Tertiärbutylthio oder Pentylthio, 3- bis 7-gliedrige Cycloalkyl-C₁-C₄-alkyl, wie Cyclopropyl- Cyclobutyl-, Cyclopentyl- oder Cyclohexylmethyl, unsubstituiertes oder durch C₁-C₄-Alkyl, wie Methyl, C₁₋C₄-Alkoxy, wie Methoxy, Hydroxy, Halogen der Atomnummer bis und mit 35, wie Chlor oder Brom, Nitro und/oder Amino substituierte Phenyl-C₁-C₄-niederalkyl bedeutet,
R' unsubstituiertes oder durch C₁-C₄-Alkyl, wie Methyl, C₁-C₄-Alkoxy, wie Methoxy, Hydroxy, Halogen der Atomnummer bis und mit 35, wie Chlor oder Brom, Nitro und/oder Amino substituierte Phenyl-C₁-C₄-niederalkyl ist,
Hal Halogen der Atomnummer bis und mit 35, wie Chlor oder Brom, darstellt und
R₃ Azido ist,
und ihre Salze.

Die Erfindung betrifft in allererster Linie Verbindungen der Formeln III, V oder VI, worin R₂ C₁-C₄-Alkyl, vorzugsweise Methyl oder verzweigtes C₁-C₄-Alkyl, wie Isopropyl, bedeutet, R₄ und R'' C₁-C₄-Alkyl, vorzugsweise Methyl oder verzweigtes C₁-C₄-Alkyl, wie Isopropyl, C₂-C₄-Alkenyl, wie Allyl oder Methallyl, oder unsubstituiertes oder durch C₁-C₄-Alkyl, wie Methyl, C₁-C₄-Alkoxy, wie Methoxy, Hydroxy, Halogen der Atomnummer bis und mit 35, wie Chlor oder Brom, Nitro und/oder Amino substituiertes Phenyl-C₁-C₄-niederalkyl bedeutet, R' Benzyl bedeutet, Hal Halogen der Atomnummer bis und mit 35, wie Chlor oder Brom, darstellt und R₃ Azido ist, und ihre Salze.

Das erfindungsgemäße Verfahren zur Herstellung von Verbindungen der Formeln II, V und VI ist dadurch gekennzeichnet, daß man
a) zur Herstellung von Verbindungen der Formel V ein 1 4-Dihalogenbuten, z.B. 1,4-Dibrombuten, zunächst mit einer Verbindung der Formel IVa und anschließend mit einer Verbindung der Formel IVb bzw. zunächst mit einer Verbindung der Formel IVb und anschließend mit einer Verbindung der Formel IVa 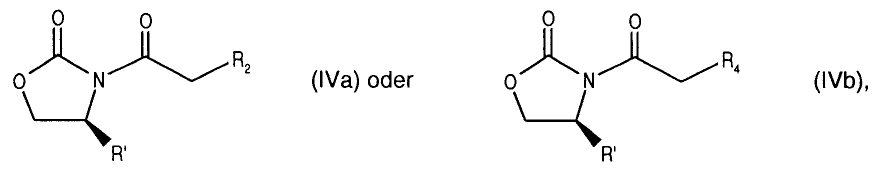 oder in einem Schritt oder schrittweise mit der etwa doppeltmolaren Menge einer Verbindung der Formel IVa oder IVb umsetzt, worin R', R₂ und R₄ die angegebenen Bedeutungen haben, oder
b) zur Herstellung von Verbindungen der Formel VI eine Verbindung der Formel V 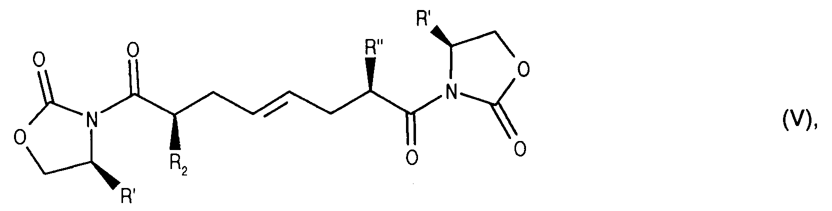 worin R'' für einen Rest R₂ oder R₄ steht, mit einem üblichen Halogenierungsmittel, wie elementarem Halogen, insbesondere Brom oder Jod, oder vorzugsweise mit einem N-Halogensuccinimid, insbesondere N-Bromsuccinimid in 1,2-Dimethoxyethan, behandelt und das gewünschte Isomere, in dem R'' für R₄ steht, abtrennt und/oder
c) zur Herstellung von Verbindungen der Formel III in einer Verbindung der Formel VI 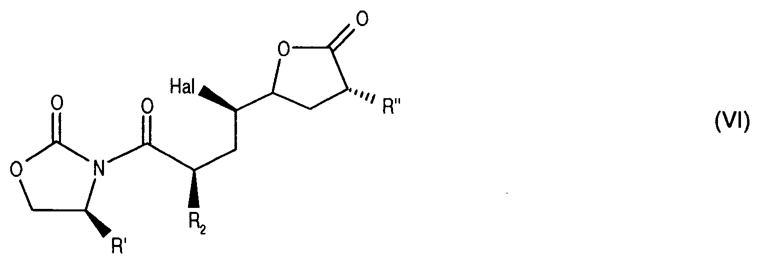 worin Hal Halogen, wie Brom, bedeutet, das Halogenatom, beispielsweise durch Behandeln mit Tetrabenzylammoniumazid in Toluol, unter Inversion durch Azido ersetzt und gewünschtenfalls die Azidogruppe zu Amino hydriert und anschließend aliphatisch oder araliphatisch substituiert und/oder durch eine Aminoschutzgruppe schützt und gewünschtenfalls eine erfindungsgemäße Verbindung der Formeln III, V oder VI in eine andere erfindungsgemäße Verbindung der Formel Formeln III, V oder VI umwandelt, ein gegebenenfalls erhältliche Isomerengemische auftrennt und/oder die verfahrensgemäß erhältliche Verbindung der Formeln III, V oder VI in ein Salz oder ein verfahrensgemäß erhältliches Salz in die freie Verbindung oder in ein anderes Salz überführt.

Die nachfolgen Beispiele dienen zur Illustration der Erfindung; Temperaturen sind in Celsiusgraden, Drucke in mbar angegeben.

Die Abkürzung "R_{f}(A)" bedeutet beispielsweise, daß der R_{f}-Wert im Lösungsmittelsystem A ermittelt wurde. Das Mengenverhältnis von Lösungsmitteln zueinander ist stets in Volumenanteilen angegeben.

Für die Bezeichnung der Fließmittel-Systeme werden bei der Flash-Chromatographie und der Mitteldruckchromatographie die gleichen Abkürzungen verwendet.

Massenspektroskopische Meßwerte werden entweder durch konventionelle MS oder nach der "Fast-Atom-Bombardment" (FAB-MS)-Methode erhalten. Die Massenangaben beziehen sich im ersten Fall auf das unprotonierte Molekülion (M)⁺ oder das protonierte Molekülion (M+H)⁺.

Die verwendeten Kurzbezeichnungen und Abkürzungen haben die folgenden Bedeutungen:
- C₁₈-Nucleosil®: Handelsname für mit Octadecylresten belegtes "Reversed Phase"- Säulenmaterial für HPLC (Nucleosil® 5C₁₈, Macherey & Nagel, BRD)
- HPLC - Säulendimension:: 250 x 4.6 mm
- HPLC - Säulenpackungen:: Nucleosil® 5C₁₈
- HPLC - Eluenten:: A) Wasser + 0.1 Vol.-% Trifluoressigsäure
B) Aceonitril + 0.1 Vol.-% Trifluoressigsäure
- HPLC - Gradient 0:: 20-100 % B in 20 Minuten + 8 Minuten 100% B
- HPLC - Gradient I:: Linear in 60 Minuten
von 30 Vol.-% B + 70 Vol.-% A
bis 90 Vol.-% B + 10 Vol.-% A
- pFAB-MS: Fast-Atom-Bombardment Mass-Spectroscopy"
- FC: "Flash-Chromatographie"
- HPLC: Hochleistungs-Flüssigchromatographie
- Hyflo®: Handelsname für Filterhilfsmittel (Fluka, Buchs, Schweiz)
- IR: Infrarotspektroskopie
- Kp: beim in Torr angegebenen Druck
- ml: Milliliter
- MS: Massenspektroskopie
- R_{f}: Verhältnis von Laufstrecke einer Substanz zu Entfernung der Laufmittelfront vom Startpunkt bei DC
- Rₜ: Retentionszeit einer Substanz bei HPLC (in Minuten)
- Schmp.: Schmelzpunkt (Temperatur).

### Beispiel 1: 2(S)-[2(S)-Azido-2(S)-(4(S)-isopropyl-5-oxo-tetrahydrofuran-2(S)-yl)-ethyl]-3- methyl-buttersäure

Zu einer bei -5° C gerührten Lösung von 55.3 g 3-{2(S)-[2(S)-Azido-2(S)-(4(S)-isopropyl-5-oxo-tetrahydrofuran-2(S)-yl)-ethyl]-3-methyl-butyryl}-4(S)-benzyl-oxazolidin-2-on in 500 mL Tetrahydrofuran werden nacheinander 175 mL Wasser, 74 mL 30%ige Wasserstoffperoxidlösung und 5.9 g Lithiumhydroxid langsam zugegeben. Das Reaktionsgemisch wird 1 Stunde bei 5° C und 150 Minuten bei Raumtemperatur weitergerührt, dann bei 3° C während 30 Minuten. mit 750 mL wäßriger 1M Natriumsulfitlösung versetzt und 30 Minuten bei Raumtemperatur weitergerührt. Durch Einengen wird dann das Reaktionsgemisch vom Tetrahydrofuran befreit und die wäßrige Lösung wird dreimal mit 1200 mL Essigsäureethylester gewaschen, wobei die organischen Phasen dreimal mit 100 mL 0.1 N Natriumhydroxid zurückextrahiert werden. Die vereinigten wäßrigen Phasen werden mit ca 200 mL 4N Salzsäure auf pH 1-2 gestellt und mit dreimal 1200 mL Essigsäureethylester extrahiert. Aus den organischen Phasen erhält man durch Vereinigen, Trocknen über Magnesiumsulfat und Eindampfen das Rohprodukt, welches zur Cyclisierung von geöffnetem Lakton in 500 mL Toluol gelöst und zusammen mit ca.1 g Molekularsieb und ca.1 g p-Toluolsulfonsäure 2 Stunden bei 50° C gerührt wird. Filtrieren, Eindampfen und Reinigen des Rückstandes mittels FC (Hexan/Essigsäureethylester/Eisessig 30: 60: 1) ergibt die Titelverbindung, welche spontan kristallisiert: Schmp. 56 -58° C; R_{f} (Hexan/Essigsäureethylester/Eisessig 30 : 60 : 1) = 0.62.

Das als Ausgangsmaterial verwendete 3-{2(S)-[2(S)-Azido-2(S)-(4(S)-isopropyl-5-oxo-tetrahydrofuran-2(S)-yl)-ethyl]-3-methyl-butyryl}-4(S)-benzyl-oxazolidin-2-on kann beispiels weise folgendermaßen hergestellt werden:

### a) trans-1,8- Bis-[4(S)-benzyl-2-oxo-oxazolidin-3-yl]-2(S),7(S)-diisopropyl-oct-4-en-1,8-dion

Zu einer Lösung von 11.5 g 4(S)-Benzyl-3-isovaleroyl-oxazolidin-2-on in 32 mL Tetrahydrofuran werden unter Rühren bei -75° C 48 mL einer 1.0 M Lösung von Lithiumhexamethyldisilazid in Tetrahydrofuran innerhalb von 1 Stunde zugetropft. Es wird noch 2 Stunden bei -75° C und 20 Minuten bei 20° C weitergerührt, dann 10 mL 1,3-Dimethyl-3,4,5,6-tetrahydro-2-(1 H)-pyrimidon (DMPU) und innert 45 Minuten die Lösung von 4.28 g 1.4-Dibrom-2-buten in 10 mL Tetrahydrofuran zugegeben. Das Reaktionsgemisch wird 15 Stunden bei -20° C weitergerührt, dann innert 1 Stunde auf 0° C gebracht, anschließend bei -20° C mit 10 mL gesättigter Ammoniumchloridlösung versetzt und nach 15 Minuten auf Raumtemperatur gebracht. Nun wird das Reaktionsgemisch zwischen Dichlormethan und gesättigter Kochsalzlösung/Wasser (1:1) verteilt. Aus den organischen Phasen erhält man nach Vereinigen, Trocknen über Natriumsulfat, Eindampfen und Reinigen des Rückstandes mittels FC (Hexan/Essigsäureethylester 4:1) die Titelverbindung: R_{f} (Hexan/Essigsäureethylester 4:1) = 0.30; Schmp. = 110-111 ° C (kristallisiert aus Essigsäureethylester/Hexan).

### b) 4(S)-Benzyl-3-{2(S)-[2(R)-brom-2(R)-(4(S)-isopropyl-5-oxo-tetrahydrofuran-2(S)-yl)-ethyl]-3-methyl-butyryl}-oxazolidin-2-on

Zu einer Lösung von 30 g *trans*-1,8-Bis-[4(S)-benzyl-2-oxo-oxazolidin-3-yl]-2(S),7(S)-diisopropyl-oct-4-en-1,8-dion in 360 mL Tetrahydrofuran und 120 mL Wasser werden unter Rühren 10.5 g N-Bromsuccinimid zugegeben, wobei die Temperatur mit einem Eisbad auf Raumtemperatur gehalten wird. Das Reaktionsgemisch wird 2 Stunden bei Raumtemperatur weitergerührt, dann wird am Rotationsverdampfer das Tetrahydrofuran abgedampft. Der wäßrige Rückstand wird zwischen Diethylether (zweimal 200 mL), Wasser (zweimal 50 mL) und gesättigter Kochsalzlösung (einmal 50 mL) verteilt. Aus den organischen Phasen erhält man nach Vereinigen, Trocknen über Magnesiumsulfat, Eindampfen und Reinigen des Rückstandes mittels FC (90 g Kieselgel, Hexan/ Essigsäureethylester 3:1) die Titelverbindung als Rohprodukt. Durch Kristallisation aus Diisopropylether erhält man die reine Verbindung: Schmp. = 91/92° C; R_{f} (Hexan/Essigsäureethylester 8:2) = 0.28.

### c) 3-{2(S)-[2(S)-Azido-2(S)-(4(S)-isopropyl-5-oxo-tetrahydrofuran-2(S)-yl)-ethyl]-3-methyl-butyryl}-4(S)-benzyl-oxazolidin-2-on

Zu einer bei Raumtemperatur gerührten Lösung von 17.8 g 4(S)-Benzyl-3-{2(S)-[2(R)-brom-2(R)-(4(S)-isopropyl-5-oxo-tetrahydrofuran-2(S)-yl)-ethyl]-3-methyl-butyryl)-oxazolidin-2-on in 180 mL Toluol werden 13.6 g frisch getrocknetes Tetrabutylammoniumazid zugegeben und im Verlauf von 160 Stunden Rühren bei Raumtemperatur werden weitere 10 g des Azids zugefügt. Danach wird das Reaktionsgemisch zwischen Essigsäureethylester und Wasser (zweimal) und gesättigter Kochsalzlösung (einmal) verteilt. Die organischen Phasen werden vereinigt, über Natriumsulfat getrocknet und eingeengt. Aus dem Eindampfrückstand erhält man mittels FC (Hexan/Essigsäureethylester 8: 2) und Kristallisation aus Diethylether/Hexan erhält man die Titelverbindung: Schmp. = 102-103° C; R_{f}(Hexan/Essigsäureethylester 8 : 2) = 0.2;.

### Beispiel 2: Alternative Herstellung von 2(S)-[2(S)-Azido-2(S)-(4(S)-isopropyl-5-oxo-tetrahydrofuran-2(S)-yl)-ethyl]-3-methyl-buttersäure

53.8 g (0.118 mol) 3-{2(S)-[2(S)-Azido-2(S)-(4(S)-isopropyl-5-oxo-tetrahydrofuran-2(S)-yl)-ethyl]-3-methyl-butyryl}-4(S)-benzyl-oxazolidin-2-on werden im Sulfierkolben in einer Mischung aus Tetrahydrofuran (215 mL) und Wasser (215 mL) gelöst und auf 0-5 °C gekühlt. Es werden unter Rühren zunächst eine 30 %-ige Wasserstoffperoxid-Lösung (73.0 mL) tropfenweise über 10 min und anschließend Lithiumhydroxid (5.60 g) in einer Portion addiert. Man läßt die weiße Suspension auf Raumtemperatur aufwärmen und rührt noch 2 Stunden nach. Das Reaktionsgemisch wird auf 0-5 °C gekühlt und eine 1 M Natriumsulfit-Lösung (600 mL) über 30 min zugetropft. Nach 30 min Rühren bleibt der Nachweis auf Peroxide (Merckoquant® Peroxid-Test) negativ. Das Reaktionsgemisch wird am Rotationsverdampfer unter Entfernung von Tetrahydrofuran eingeengt und die alkalische wäßrige Phase (ca. 900 mL) anschließend mit Essigsäureethylester (3 x 200 mL) extrahiert. Die vereinten organischen Phasen werden mit 0.1 N Natronlauge (1 x 30 mL) rückextrahiert, mit gesättigter Natriumchlorid-Lösung (1 x 60 mL) gewaschen, über Magnesiumsulfat getrocknet, filtriert und eingeengt. Man erhält das rohe 4(S)-Benzyl-1,3-oxazolidin-2-on als weiß-beige Festsubstanz (23.0 g roh, >100 %). Die im ersten Teil der Aufarbeitung erhaltenen alkalisch wäßrigen Phasen werden vereint, durch Zugabe von 4 N Salzsäure auf pH 1-2 gestellt, mit Essigsäureethylester (4 x 250 mL) extrahiert und die organischen Phasen mit gesättigter Natriumchlorid-Lösung (1 x 60 mL) gewaschen, über Magnesiumsulfat getrocknet, filtriert und eingeengt. Das so erhaltene blaßgelbe Öl (48.3 g) wird in Toluol (500 mL) gelöst, mit einer katalytischen Menge p-Toluolsulfonsäure-Monohydrat (110 mg) versetzt und über 20 min bei 40 °C gerührt. Die klare Lösung wird am Rotationsverdampfer eingeengt und der Rückstand über Nacht im Hochvakuum getrocknet. Man erhält die rohe Titelverbindung als blaßgelbes Öl (34.0 g, 97 %), das bei Raumtemperatur langsam kristallisiert. R₁ (Hexan/Essigsäureethylester/Eisessig 50:50:1) = 0.44.

Das als Ausgangsmaterial verwendete 3-{2(S)-[2(S)-Azido-2(S)-(4(S)-isopropyl-5-oxo-tetrahydrofuran-2(S)-yl)-ethyl]-3-methyl-butyryl}-4(S)-benzyl-oxazolidin-2-on kann in einer Abwandlung der Verfahrens gemäß Beispiel 1 auch folgendermaßen hergestellt werden:

### a) trans-1,8-Bis-[4(S)-benzyl-2-oxo-oxazolidin-3-yl]-2(S),7(S)-diisopropyl-oct-4-en-1,8-dion

Zur Lösung von 4(S)-N-Isovaleroyl-4-benzyl-oxazolidin-2-on (10.0 g) in Tetrahydrofuran (26 mL) wird bei 0 °C unter Argon eine 1 M Lithiumhexamethyldisilazan-Lösung (39.2 mL) in Tetrahydrofuran über 1 h zugetropft, wobei die Reaktionstemperatur unterhalb von 3 °C gehalten wird. Nach beendeter Zugabe wird die Mischung über 2 h bei 0 °C nachgerührt. Anschließend werden 1,3-Dimethyl-3,4,5,6-tetrahydro-2(1*H*)-pyrimidinon (9.4 mL) und eine Lösung von *trans*-1,4-Dibrom-2-buten (4.0 g) in Tetrahydrofuran (15.4 mL) addiert, die Lösung während 18 h bei 0 °C gerührt und schließlich die Reaktion durch langsame Zugabe einer gesättigten wäßrigen Ammoniumchlorid-Lösung (30 mL) gequencht. Man verdünnt mit Diethylether (500 ml), wäscht die organische Phase mit Wasser (2 x 100 mL), extrahiert die wäßrigen Phasen mit Diethylether (2 x 100 mL) zurück, wäscht die vereinten organischen Phasen mit gesättigter Natriumchlorid-Lösung (2 x 100 mL), trocknet über Natriumsulfat und engt im Vakuum ein. Das Rohprodukt (14.9 g) wird aus Methanol umkristallisiert, und man erhält die Titelverbindung (8.41 g) als weißes Pulver. Schmp. 108-109 °C (Schmp. 110-111 °C aus Diethylether/Hexan). [α]²⁵_{D} = +75.5 ± 1.0 (*c*1, CH₂Cl₂). ¹H-NMR (500 MHz, DMSO-d₆): δ 0.84 (d, J = 6.8 Hz, 6H), 0.87 (d, J = 6.8 Hz, 6H), 1.87 (m, 2H), 2.23 (m, 2H), 2.34 (m, 2H), 2.84 (dd, J = 8.2, 13.5 Hz, 2H), 3.01 (dd, J = 3, 13.5 Hz, 2H), 3.66 (m, 2H), 4.13 (dd, J = 2.7, 8.8 Hz, 2H), 4.30 (t, J = 8.5 Hz, 2H), 4.67 (m, 2H), 5.46 (m, 2H), 7.2-7.3 (m, 10 H) ppm. Anal. C₃₄H₄₂N₂O₆ (574.72): ber. C= 71.06%, H= 7.37%, N= 4.87%; gef. C= 71.20%; H= 7.40%; N= 4.90%.

### b) 4(S)-Benzyl-3-{2(S)-[2(R)-brom-2(R)-(4(S)-isopropyl-5-oxo-tetrahydrofuran-2(S)-yl)-ethyl]-3-methyl-butyril}-oxazolidin-2-on

Das nach der in a) beschriebenen Methode erhaltene Produkt (50.0 g) wird in Dichlormethan (1 L) gelöst. Nach Zugabe von Wasser (250 mL) wird das zweiphasige Gemisch bei Raumtemperatur während 30 min gerührt. Dann wird N-Bromsuccinimid (17 g) in einer Portion zugegeben und der Ansatz 15 min verrührt. Anschließend wird auf 35 °C erwärmt und bei dieser Temperatur während 24 Stunden gut gerührt. Nach dem Abkühlen auf Raumtemperatur werden die beiden Phasen getrennt, die wäßrige Phase mit Dichlormethan (200 mL) extrahiert und die vereinten organischen Phasen mit 0.1 N Natriumthiosulfat-Lösung (250 mL) und Wasser (250 mL) gewaschen. Die wäßrigen Phasen werden jeweils einmal mit Essigsäureethylester rückextrahiert. Nach dem Einengen des Lösungsmittels und Trocknen im Hochvakuum erhält man das Rohprodukt als leicht gelbliches, zähflüssiges Öl (62 g). Umkristallisation aus einem 4:1-Gemisch Isopropanol/Wasser (200 mL) ergibt die Titelverbindung als weiße kristalline Festsubstanz (33 g, 77 %). Schmp. 91-93 °C. [α]²⁵_{D} = +38.6±1.0 (*c*1, CH₂Cl₂). ¹H-NMR (500 MHz, DMSO-d₆): δ 0.85 (d, J = 6.8 Hz, 3H), 0.86 (d, J = 6.9 Hz, 3H), 0.93 (d, J = 6.9 Hz, 3H), 0.95 (d, J = 6.9 Hz, 3H), 1.93 (m, 1H), 2.01 (m, 1H), 2.1-2.3 (m, 4H), 2.72 (m, 1H), 2.83 (dd, J=8.8, 13.4 Hz, 1H), 3.14 (dd, J=3, 13.4 Hz, 1H), 3.89 (m, 1H), 4.15 (dd, J = 2.4, 8.8 Hz, 1H), 4.31 (t, J = 8.6 Hz, 1H), 4.40 (m, 1H), 4.6-4.7 (m, 2H), 7.2-7.3 (m, 5H) ppm. Anal. C₂₄H₃₂BrNO₅ (494.43): ber. C= 58.30%, H= 6.52%, N= 2.83%, Br= 16.16%; gef. C= 58.09%; H= 6.61%; N= 2.72%, Br= 16.21%.

Die anfallende Mutterlauge wird im Vakuum bei 50 °C unter azeotroper Entfernung von restlichem Wasser eingeengt. Der erhaltene ölige Rückstand (25 g) wird unter Rühren bei Raumtemperatur mit Methyl-tert.-butylether (125 ml) versetzt, wobei Kristallisation einsetzt. Nach 30 min wird Hexan (200 mL) zugesetzt und noch weitere 30 min bei Raumtemperatur gerührt. Der Niederschlag wird abfiltriert, mit einem 1:2-Gemisch Methyl-tert.-butylether/Hexan (15 mL) gewaschen und getrocknet. Man erhält das 4(S)-Benzyl-1,3-oxazolidin-2-on als weiße kristalline Festsubstanz (13.6 g, 88 %) vom Schmp. 86-88 °C.

### c1) 3-{2(S)-[2(S)-Azido-2(S)-(4(S)-isopropyl-5-oxo-tetrahydrofuran-2(S)-yl)-ethyl]-3-methyl-butyryl}-4(S)-benzyl-oxazolidin-2-on

Das nach der in b) beschriebenen Methode erhaltene Produkt (66.0 g) und Tricapryl-ylmethylammoniumchlorid (2.67 g) werden in Toluol (1 L) gelöst und unter Rühren mit einer Lösung von Natriumazid (34.7 g) in Wasser (100 mL) versetzt. Die Emulsion wird bei 76 °C (Ölbad 100 °C) während 48 Stunden gerührt und anschließend auf Raumtemperatur abgekühlt. Nach Trennung der beiden Phasen wird die wäßrige Phase mit Toluol (100 mL) extrahiert und die vereinten organischen Phasen mit Wasser (2 x 100 mL) gewaschen. Einengen des Lösungsmittels und Trocknen im Hochvakuum ergibt das Rohprodukt (70.1 g) als gelbliches Öl. Nach Auskristallisation aus einem 1:1-Gemisch Methyl-*tert*-butylether/Hexan (560 mL) wird der Niederschlag filtriert, mit einem eiskalten 1:1-Gemisch Methyl-tert.-butyl-ether/Hexan (2 x 15 mL) gewaschen und bei 50 °C im Hochvakuum getrocknet. Man erhält die Titelverbindung als weiße kristalline Festsubstanz (46 g, 75 %). Schmp. 104-105 °C. IR (KBr) 2980, 2100, 1775 (breite Doppelbande), 1695, 1385, 1345, 1190 cm cm⁻¹. ¹H-NMR (500 MHz, DMSO-d₆): δ 0.82 (d, J = 6.9 Hz, 3H), 0.87 (d, J = 6.8 Hz, 3H), 0.92 (d, J = 6.8 Hz, 3H), 0.95 (d, J = 6.8 Hz, 3H), 1.52 (m, 1H), 1.94 (m, 1H), 1.95-2.1 (m, 2H), 2.18 (m, 1H), 2.66 (m, 1H), 2.90 (dd, J =8.3, 13.4 Hz, 1H); 3.10 (dd, J = 3.4, 13.4 Hz, 1H), 3.38 (m, 1H), 3.86 (m, 1H), 4.17 (dd, J =2.4, 8.8 Hz, 1H), 4.34 (t, J = 8.6 Hz, 1H), 4.34 (m,1H), 4.48 (m, 1H), 4.71 (m, 1H), 7.2-7.3 (m, 5H) ppm. [α]²⁵_{D} = +35.2 ± 1.0 (*c*1, CH₂Cl₂). Anal. C₂₄H₃₂N₄O₅ (456.54): ber. C= 63.14%, H= 7.06%, N= 12.27%; gef. C= 62.94%; H= 7.12%; N= 12.08%.

### c2) Alternative Herstellung von 3-{2(S)-[2(S)-Azido-2(S)-(4(S)-isopropyl-5-oxo-tetrahydrofuran-2(S)-yl)-ethyl]-3-methyl-butyryl}4(S)-benzyl-oxazolidin-2-on

Zu einer Lösung des Produkts aus b) (1.0 g) in Toluol (15 mL) wird bei Raumtemperatur Tetrabutylammoniumazid (0.69 g; hergestellt nach dem von M. De Giorgi et al. in Synth.Commun. (1987), 17, 521-533 beschriebenen Verfahren) addiert und der Ansatz bei 50 °C über 12 h gerührt. Das Reaktionsgemisch wird nach dem Abkühlen mit Essigsäureethylester verdünnt, die organische Phase mit Wasser gewaschen, über Natriumsulfat getrocknet und eingeengt. Nach Umkristallisation des Rückstands aus Diisopropylether erhält man die Titelverbindung als kristallinen Festkörper (0.51g, 56 %).

### Beispiel 3: Alternative Herstellung von 2(S)-[2(S)-Azido-2(S)-(4(S)-isopropyl-5-oxo-tetrahydrofuran-2(S)-yl)-ethyl]-3-methyl-buttersäure

Zu einer Lösung des Produktes aus Beispiel 2c) (50.0 g) in Tetrahydrofuran (250 mL) und Wasser (125 mL) wird bei 0-5 °C unter Rühren eine 30%ige Wasserstoffperoxid-Lösung (67 mL) über 15 min zugetropft und anschließend Lithiumhydroxid (7.85 g) in einer Portion addiert. Man läßt auf Raumtemperatur erwärmen und weitere 3 h nachrühren. Das Reaktionsgemisch wird auf 5-10 °C gekühlt, und solange tropfenweise mit einer 2 M Natriumsulfit-Lösung versetzt, bis der Nachweis auf Peroxid (Merckoquant® Peroxid-Test) negativ bleibt (190 mL einer 2 M Natriumsulfit-Lösung). Der Reaktionsansatz wird mit Essigsäureethylester (3 x 100 mL) gewaschen und die vereinte organische Phase mit 0.1 N Natronlauge rückextrahiert. Die vereinte alkalisch wäßrige Phase wird mit 4 N Salzsäure auf pH 4-5 gestellt und mit Essigsäureethylester (3 x 100 mL) extrahiert. Die vereinten organischen Phasen werden mit Toluol (100 mL) versetzt und anschließend langsam am Rotavap eingeengt. Man erhält die rohe Titelverbindung als farbloses Öl (31.6 g, 97 %). Umkristallisation aus Hexan/Diethylether ergibt ein analysenreines Produkt: Schmp. 58-60 °C. IR (CH₂Cl₂) 3500 (w), 2965, 2110, 1775, 1745 (w), 1705, 1180 cm⁻¹· [α]²⁵_{D} = -23.4 ± 1.0 (*c*1, CH₂Cl₂). ¹H-NMR (300 MHz, CD₃OD): δ 0.92 (d, J = 6.9 Hz, 3H), 0.95 (d, J = 6.8 Hz, 6H), 1.04 (d, J = 6.9 Hz, 3H), 1.64 (m, 1H), 1.85 (m, 1H), 1.95 (m, 1H), 2.05-2.3 (m, 3H), 2.42 (ddd, J = 3, 6.5, 11.6 Hz, 1H), 2.70 (ddd, J = 5.2, 6.8, 10.2 Hz, 1H), 3.36 (ddd, J = 2.6, 5.6, 11.1 Hz, 1 H), 4.48 (ddd, J = 5.6, 5.6, 8.2 Hz, 1H) ppm. C₁₄H₂₃N₃O₄ (297.36): ber. C= 56.54%, H= 7.79%, N= 14.13%; gef. C= 56.76%; H= 7.71%; N= 13.81%.

### Beispiel 4:2-{2(S)-Azido-2-[4(S)-isopropyl-5-oxo-tetrahydrofuran-2(S)-yl]-ethyl}-3(S)-methyl-butyraldehyd

Zu einer Lösung von 1.7 g 2(S)-[2(S)-Azido-2(S)-(4(S)-isopropyl-5-oxo-tetrahydrofuran-2(S)-yl)-ethyl]-3-methyl-buttersäure in 20 mL Toluol werden bei 0° C unter Rühren 1.45 mL Oxalylchlorid innert 10 Minuten. zugetropft. Danach werden noch 0.03 mL n;n-Dimethylformamid zugegeben und die Temperatur innert 30 innerhalb von 30 Minuten auf 37° C erhöht. Das Reaktionsgemisch wird 2 Stunden bei 37° C gerührt, dann klarfiltriert und unter vermindertem Druck bei 30° C Badtemperatur vorsichtig stark eingeengt (vollständiges Eindampfen vermeiden!) eingedampft. Der Rückstand wird noch zweimal in 10 mL Toluol gelöst und in gleicher Weise wieder eingeengt. Das so erhaltene rohe Säurechlorid wird in 5 mL Tetrahydrofuran gelöst und bei -75° C innerhalb von 30 Minuten mit 16 mL einer 0.34 M Lösung von NaAlH(O-tert.Bu)₃ in Diglyme versetzt. Das Reaktionsgemisch wird 70 Minuten. bei -75° C weitergerührt und dann bei gleicher Temperatur die Lösung von 0.385 mL Eisessig in 1 mL Tetrahydrofuran zugetropft, gefolgt von 2.1 mL gesättigter Ammoniumchloridlösung und 20 mL Diethylether. Der Ansatz wird auf Raumtemperatur gebracht und zwischen Diethylether und Wasser/gesättigter Kochsalzlösung verteilt. Aus den organischen Phasen erhält man nach Vereinigen, Trocknen über Magnesiumsulfat, Eindampfen und Reinigen des Rückstandes mittels FC (Hexan/Essigsäureethylester = 95 : 5) die Titelverbindung: R_{f} (Hexan/Essigsäureethylester = 2 : 1) = 0.55; HPLC Rₜ = 16.4 Minuten.

### Beispiel 5: 2-{2(S)-Azido-2-[4(S)-isopropyl-5-oxo-tetrahydrofuran-2(S)-yl]-ethyl}-3(S)-methyl-butanol

Zur Lösung von 8.0 g 2(S)-[2(S)-Azido-2(S)-(4(S)-isopropyl-5-oxo-tetrahydrofuran-2(S)-yl)-ethyl]-3-methyl-buttersäure (Rohprodukt aus Beispiel 2 oder 3) in wasserfreiem Tetrahydrofuran (180 mL) wird bei -10 °C nacheinander Triethylamin (5.62 mL) und Chlorameisensäuremethylester (2.59 mL) zugetropft. Die weiße Suspension wird zunächst bei -10 °C über 1 h und anschließend bei 0 °C über 2 h nachgerührt. Der Ansatz wird mit Essigsäureethylester (100 mL) verdünnt und die organische Phase nacheinander mit eiskalter 0.5 N Salzsäure, gesättigter Natriumhydrogencarbonat-Lösung und Wasser gewaschen, über Natriumsulfat getrocknet und eingeengt. Der blaßgelbe ölige Rückstand wird in Tetrahydrofuran (160 mL) aufgenommen, und unter Rühren bei -20 °C portionsweise Natriumborhydrid (1.12 g) addiert. Anschließend wird über 10 min Methanol (1.5 mL) zugetropft (geringe Exothermie). Man läßt die leicht trübe Mischung langsam auf 0-5 °C aufwärmen und bei dieser Temperatur über Nacht weiterrühren, tropft anschließend 1 N Salzsäure (39 mL) zu und extrahiert die wäßrige Phase mit Essigsäureethylester (100 mL). Die organische Phase wird mit eiskalter 1 N Natriumcarbonat-Lösung (70 mL) und dann mit gesättigter Natriumchlorid-Lösung neutral gewaschen, über Magnesiumsulfat getrocknet und eingeengt. Nach Trocknen im Hochvakuum erhält man die Titelverbindung als blaßgelbes Öl (7.18 g, 94 %). Analysenreines Produkt (blaßgelbes Öl) erhält man nach Flash-Chromatographie an Kieselgel (Fließmittel-Gradient Hexan/Essigsäureethylester von 5:1 nach 3:1): R_{f} (Hexan/Essigsäureethylester 1:1) = 0.50. [α]²⁵_{D} = + 4.6 ± 1.0 (*c* 1.0, CH₂Cl₂). IR (CH₂Cl₂) 3620, 2960, 2110, 1770, 1180 cm⁻¹.¹H-NMR (300 MHz, CDCl₃): δ0.92 (d, J = 6.9 Hz, 3H), 0.95 (d, J = 6.8 Hz, 6H), 1.04 (d, J = 6.9 Hz, 3H), 1.35-1.8 (m, 5H), 2.1-2.25 (m, 3H), 2.69 (m, 1H), 3.57 (m, 1H), 3.67 (m, 1H), 3.72 (m, 1H), 4.44 (m, 1H)ppm. ¹³C-NMR (75 MHz, CDCl₃): δ 19.4, 20.2, 21.1, 21.3, 28.0, 30.2, 30.3, 31.1, 43.8, 46.3, 64.9, 65.1, 81.8, 179.4 ppm. C₁₄H₂₅N₃O₃(283.4): ber. C= 59.34%, H= 8.89%, N= 14.83%; gef. C= 59.35%; H= 8.85%; N=, 14.41%.

### Beispiel 6: Alternative Herstellung von 2-{2(S)-Azido-2-[4(S)-isopropyl-5-oxo- tetrahydrofuran-2(S)-yl]-ethyl}-3(S)-methyl-butanol

Zur Lösung von 4.0 g 2(S)-[2(S)-Azido-2(S)-(4(S)-isopropyl-5-oxo-tetrahydrofuran-2(S)-yl)-ethyl]-3-methyl-buttersäure (Rohprodukt aus Beispiel 2 oder 3) in Toluol (80 mL) wird bei 0-5 °C Chlorameisensäureethylester (1.38 mL) über 10 min zugetropft und der Ansatz 20 min bei gleicher Temperatur nachgerührt. Die weiße Suspension wird über eine Glasfritte filtriert, der Niederschlag mit Toluol (30 mL) nachgewaschen und das Filtrat am Rotavap (Badtemperatur 35 °C) auf ein Volumen von 40 mL eingeengt. Anschließend verdünnt man die klare Lösung mit Tetrahydrofuran (80 mL), kühlt auf 0-5 °C und addiert nacheinander unter kräftigem Rühren Natriumborhydrid (0.68 g) und Wasser (1.5 mL). Nach 3 h wird erneut Natriumborhydrid (0.10 g) zugefügt und der Ansatz eine weitere Stunde bei 0-5 °C gerührt. Man versetzt unter Eiskühlung mit 1 N Salzsäure (30 mL), extrahiert die wäßrige Phase mit Essigsäureethylester (1 x 100 mL) und wäscht die organische Phase nacheinander mit 1 N Natriumcarbonat-Lösung (2 x 20 mL) und gesättigter Natriumchlorid-Lösung (3 x 20 mL). Die wäßrigen Phasen werden jeweils mit kaltem Essigsäureethylester rückextrahiert. Die vereinten organischen Phasen werden über Magnesiumsulfat getrocknet und eingeengt. Nach dem Trocknen im Hochvakuum erhält man die Titelverbindung als blaßgelbes Öl (3.47 g, 92 %).

### Beispiel 7: 2-{2(S)-tert-Butoxycarbonylamino-2-[4(S)-isopropyl-5-oxo-tetrahydrofuran-2(S)-yl]-ethyl}-3(S)-methyl-butanol

Die Lösung von 24.8 g 2-{2(S)-Azido-2-[4(S)-isopropyl-5-oxo-tetrahydrofuran-2(S)-yl]-ethyl}-3(S)-methyl-butanol (Produkt aus Beispiel 5 oder 6) in Essigsäureethylester (250 mL) wird in Gegenwart von 10 % Pd/C (8.68 g) bei Raumtemperatur und Normaldruck über 24 h hydriert. Man filtriert über Hyflo®, wäscht mehrmals mit Essigsäureethylester nach und engt ein. Das so erhaltene rohe 5(S)-[1(S)-Amino-3(S)-hydroxymethyl-4-methylpentyl]-3(S)-isopropyl-dihydrofuran-2-on (23.0 g; farbloses Öl, das langsam zu einem wachsartigen weißen Festkörper auskristallisiert) wird in Essigsäureethylester (500 mL) gelöst und bei 0-5 °C unter Rühren zunächst mit Ethyldiisopropylamin (23.7 mL) und dann tropfenweise mit einer Lösung von Di-*tert*-butyldicarbonat (21.0 g) in Essigsäureethylester (100 mL) versetzt. Nach Aufwärmen auf Raumtemperatur läßt man über Nacht weiterrühren. Das Reaktionsgemisch wird eingeengt und der ölige Rückstand durch Flash-Chromatographie an 250 g Kieselgel (2:1 Hexan/Essigsäureethylester als Eluens) gereinigt. Man erhält die Titelverbindung als weiße Festsubstanz (24.9 g, 80 %). Schmp.126-128 °C (umkristallisiert aus Diethylether). ¹H-NMR (300 MHz, CDCl₃): δ 0.86 (d, J = 6.8 Hz, 3H), 0.90 (d, J = 6.9 Hz, 3H), 0.94 (d, J = 6.8 Hz, 3H), 1.01 (d, J = 6.8 Hz, 3H), 1.35-1.7 (m, 3H), 1.44 (s, 9H), 1.74 (m, 1H), 1.96 (m, 1H), 2.05-2.25 (m, 3H), 2.56 (m, 1H), 3.60 (m, 1H), 3.69 (m, 1H), 3.99 (m, 1H), 4.4-4.55 (m, 2H) ppm. [α]²⁵_{D} = -14.9 (*c* 1.01, CH₂Cl₂). C₁₉H₃₅NO₅ (357.49): ber. C= 63.84%, H= 9.87%, N= 3.92%; gef. C= 63.62%; H= 9.66%; N= 4.05%.

### Beispiel 8: Dicyclohexylammmoniumsalz von 2(S)-[2(S)-Azido-2(S)-(4(S)-isopropyl-5-oxo-tetrahydrofuran-2(S)-yl)-ethyl]-3-methyl-buttersäure

Zur Lösung von 297 mg 2(S)-[2(S)-Azido-2(S)-(4(S)-isopropyl-5-oxo-tetrahydrofuran-2(S)-yl)-ethyl]-3-methyl-buttersäure (Produkt aus Beispiel 2 oder 3) in Methanol (5 mL) wird Dicyclohexylamin (182 mg) bei Raumtemperatur addiert. Man engt die Mischung am Rotavap ein und trocknet den weißen Rückstand im Hochvakuum. Anschließend wird das rohe Salz in Essigsäureethylester (8 mL) in der Wärme gelöst, klarfiltriert und unter Rühren mit Hexan (8 mL) versetzt. Man läßt auf Raumtemperatur abkühlen, filtriert die ausgefallenen Kristalle ab, wäscht mit 3:1 Hexan/Essigsäureethylester nach und trocknet am Hochvakuum bei 50 °C. Man erhält die Titelverbindung als weißen kristallinen Festkörper (360 mg, 75 %). Schmp. 139-140 °C.¹H-NMR (10:1 C₆D₆/DMSO-d₆): δ 0.73 (d, J = 7 Hz, 3H), 0.78 (d, J = 7 Hz, 3H), 0.85 (d, J = 7 Hz, 3H), 0.92 (d, J = 7 Hz, 3H), 0.9-1.2 (m, 11H), 1.43 (m, 2H), 1.5-1.6 (m, 5H), 1.65-1.9 (m, 7H), 1.94 (m, 1H), 2.36 (m, 1H), 2.4-2.55 (m, 3H), 3.41 (m, 1H), 3.2-4.1 (br s, 2H), 4.17 (m, 1H) ppm. C₂₆H₄₆N₄O₄ 0.16 H₂O (481.6): ber. C= 64.85%, H= 9.70%, N= 11.63%; gef. C= 65.14%; H= 9.76%; N= 11.47%.

### Beispiel 9: Weiterumsetzung von 2-{2(S)-Azido-2-[4(S)-isopropyl-5-oxo-tetrahydrofuran-2(S)-yl]-ethyl)-3(S)-methyl-butyraldehyd zu 5(S)-Amino-4(S)-hydroxy-2(S)-isopropyl-7(S)-[4-methoxy-3-(2-methoxymethoxyethyl)-benzyl]-8-methyl-nonansäure-(N-butyl)amid

### 1) 5(S)-{{1(S)-azido-3(S)-{(R,S)-hydroxy-[4-methoxy-3-(2-methoxymethoxyethyl)phenylmethyl}-4-methyl-pentyl}}3(S)-isopropyl-tetrahydrofuran-2-on

Eine Suspension von 763 mg Magnesiumspänen in 0.5 mL Tetrahydrofuran wird mit einigen Jodkristallen versetzt und 30 Minuten im Ultraschallbad aktiviert. Danach werden 4 Tropfen 1,2-Dibromethan und anschließend eine Lösung von 8.64 g 4-Brom-2-(2-methoxymethoxyethyl)-anisol in 30 mL Tetrahydrofuran derart zugetropft, daß das Reaktionsgemisch am Rückfluß siedet. Nach beendeter Zugabe wird noch 1 Stunde unter Rückfluß gehalten. Dieses Reaktionsgemisch wird nach Abkühlen auf Raumtemperatur innerhalb von 45 Minuten zu einer auf -75° C gekühlten Lösung von 2.85 g 2-{2(S)-Azido-2-[4(S)-isopropyl-5-oxo-tetrahydrofuran-2(S)-yl]-ethyl}-3(S)-methyl-butyraldehyd in 20 mL Tetrahydrofuran unter Rühren zugetropft. Das Reaktionsgemisch wird noch 150 Minuten bei -75° C weitergerührt und dann bei der gleichen Temperatur mit der Lösung von 1.4 mL Eisessig in 1 mL Tetrahydrofuran und danach mit 25 mL gesättigter Ammoniumchloridlösung versetzt. Anschließend wird der Ansatz auf Raumtemperatur gebracht, auf 60 mL Wasser gegossen und dreimal mit 100 mL Essigsäureethylester extrahiert. Die organischen Phasen werden mit 50 mL gesättigter Kochsalzlösung gewaschen, vereinigt, über Magnesiumsulfat getrocknet und eingedampft. Durch Reinigen des Rückstandes mittels FC (400 g Kieselgel, Hexan/Essigsäureethylester 8:2) erhält man die Titelverbindung: R_{f} (Hexan/Essigsäureethylester 7:3) = 0.25; HPLC Rₜ = 48.1 und 50.3 Minuten;. (Diastereomerengemisch).

### 2) {2(S)-{2(S)-Azido-2-[4(S)-isopropyl-5-oxo-tetrahydrofuran-2(S)-yl]-ethyl}-3-methyl-1(R,S)- [4-methoxy-3-(2-methoxymethoxyethyl)-phenyl]-butyl}-isobutyrat

Eine Lösung von 300 mg 5(S)-{{1(S)-azido-3(S)-{(R,S)-hydroxy-[4-methoxy-3-(2-methoxymethoxyethyl)phenyl-methyl}-4-methyl-pentyl}}-3(S)-isopropyl-tetrahydrofuran-2-on in 3.5 mL Dichlormethan wird mit 0.25 mL Pyridin, 0.31 mL Isobuttersäureanhydrid und 15 mg Dimethylaminopyridin versetzt und 80 Stunden bei Raumtemperatur weitergerührt. Danach wird das Reaktionsgemisch zwischen Dichlormethan (dreimal), Wasser (einmal) und gesättigter Kochsalzlösung (zweimal) verteilt. Aus den vereinigten organischen Phasen erhält man nach Trocknen über Magnesiumsulfat, Eindampfen und Reinigen des Rückstandes durch FC (30 g Kieselgel, Hexan/Essigsäureethylester 8:2) die Titelverbindung: R_{f} (Hexan/Essigsäureethylester 8:2) = 0.26; HPLC Rₜ = 21.4 Minuten. und 21.8 Minuten. (Diastereomerengemisch).

### 3) {4(S)-Azido-7(S)-butylcarbamoyl-5(S)-hydroxy-2(S)-isopropyl-8-methyl-1(R,S)-[4-methoxy-3-(2-methoxymethoxyethyl)-phenyl]-nonyl}-isobutyrat

Eine Lösung von 170 mg {2(S)-(2(S)-Azido-2-[4(S)-isopropyl-5-oxo-tetrahydrofuran-2(S)-yl]-ethyl}-3-methyl-1(R,S)-[4-methoxy-3-(2-methoxymethoxyethyl)-phenyl]-butyl}-isobutyrat in 1.4 mL Butylamin wird 16 Stunden bei Raumtemperatur gerührt und dann eingedampft. Aus dem Rückstand erhält man nach Reinigung mittels FC (Hexan/Essigsäureethylester 7:3) die Titelverbindung: R_{f} (Hexan Essigsäureethylester 7:3) = 0.25; HPLC Rₜ = 20.38 und 20.8 Minuten. (Diastereomerengemisch).

### 4) 5(S)-Amino-4(S)-hydroxy-2(S)-isopropyl-7(S)-[4-methoxy-3-(2-methoxymethoxyethyl)-benzyl]-8-methyl-nonansäure-(N-butyl)amid

50 mg {4(S)-Azido-7(S)-butylcarbamoyl-5(S)-hydroxy-2(S)-isopropyl-8-methyl-1(R,S)-[4-methoxy-3-(2-methoxymethoxyethyl)-phenyl]-nonyl}-isobutyrat werden in 10 mL Methanol in Gegenwart von 50 mg Pd/C 10% bei Raumtemperatur und Normaldruck hydriert. Das Reaktionsgemisch wird filtriert und eingedampft. Der Rückstand wird mittels FC (2 g Kieselgel, Dichlormethan/Methanol 9:1) gereinigt. Man erhält die Titelverbindung: R_{f} (Dichlormethan/Methanol 9:1) = 0.19; HPLC Rₜ = 13.42 Minuten.; FAB-MS (M+H)+ = 509.

Das als Ausgangsmaterial verwendete 4-Brom-2-(2-methoxymethoxyethyl)-anisol wird wie folgt hergestellt:

### a) 2-(2-Hydroxyethyl)-anisol

Zu einer Lösung von 10 g 2-(2-Hydroxyphenyl)-ethanol in 200 mL Aceton werden 35.3 g Caesiumcarbonat und anschließend die Lösung von 6.5 mL Methyljodid in 40 mL Aceton zugegeben. Das Reaktionsgemisch wird 50 Minuten bei Raumtemperatur gerührt, filtriert und eingedampft. Der Rückstand wird zwischen Diethylether und Wasser verteilt. Aus den organischen Phasen erhält man nach Vereinigen, Trocknen über Magnesiumsulfat, Eindampfen und Reinigen des Rückstandes mittels FC (Dichlormethan-Diethylether 97: 3) die Titelverbindung: R_{f} (Dichlormethan/Diethylether 97: 3) = 0.34; HPLC Rₜ = 9.31 Minuten.

### b) 4-Brom-2-(2-hydroxyethyl)-anisol

Eine Lösung von 10.7 g 2-(2-Hydroxyethyl)-anisol in 195 mL Dichlormethan und 130 mL Methanol wird portionenweise mit 35.72 g Tetrabutylammoniumtribromid versetzt. Das Reaktionsgemisch wird 150 Minuten bei Raumtemperatur gerührt und dann am Rotavapor eingedampft. Der Rückstand wird zwischen Diethylether und Wasser verteilt. Aus den organischen Phasen erhält man durch Vereinigen, Trocknen über Magnesiumsulfat, Eindampfen und Reinigen des Rückstandes mittels FC (Dichlormethan) die Titelverbindung: R_{f}(Dichlormethan) = 0.26; HPLC Rₜ = 13.0 Minuten.

### c) 4-Brom-2-(2-methoxymethoxy-ethyl)-anisol

Eine Lösung von 948 mg 4-Brom-2-(2-hydroxyethyl)-anisol in 30 mL Dichlormethan wird bei Raumtemperatur mit 1.48 g N-Ethyl-diisopropylamin und 0.49 g Chlordimethyläther versetzt. Das Reaktionsgemisch wird 200 Minuten bei Raumtemperatur weitergerührt und dann mit 1 mL Wasser und 1 mL 25%iger Ammoniumhydroxidlösung versetzt. Das Zweiphasengemisch wird 15 Minuten kräftig weitergerührt und dann wird die organische Phase abgetrennt, über Natriumsulfat getrocknet und eingedampft. Aus dem Rückstand erhält man nach Reinigung mittels FC (Hexan/Dichlormethan 1: 1) die Titelverbindung: R_{f} (Dichlormethan) = 0.6; HPLC Rₜ= 17.33 Minuten.

### Beispiel 10: Weiterumsetzung von 2-{2(S)-Azido-2-[4(S)-isopropyl-5-oxo-tetrahydrofuran-2(S)-yl]-ethyl}-3(S)-methyl-butyraldehyd zu 5(S)-Amino-4(S)-hvdroxy-2(S)-isopropyl-7(S)-[4-(3-hydroxypropoxy)-3-(3-methoxypropoxy)-benzyl]-8-methyl-nonansäure-N-(2-morpholin-4-yl-ethyl)amid-hydrochlorid

### 1) 5(S)-{1(S)-Azido-3(S)-{(R,S)-hydroxy-[4-(3-benzyloxypropoxy)-3-(3-methoxypropoxy)-phenyl-methyl}-4-methyl-pentyl}-3(S)-isopropyl-tetrahydrofuran-2-on

Zu einer bei -75° C gerührten Lösung von 500 mg 4-(3-Benzyloxypropoxy)-3-(3-methoxypropoxy)-brombenzol in 2 mL Tetrahydrofuran werden 1.3 mL einer 0.9 M Lösung von Butyllithium in Hexan langsam zugetropft. Das Reaktionsgemisch wird 20 Minuten bei -75° C weitergerührt und dann mit einer aus 44.5 mg Magnesiumpulver und 0.158 mL 1,2-Dibromethan in 3 mL Tetrahydrofuran bei Raumtemperatur frisch hergestellten Suspension von Magnesiumbromid tropfenweise versetzt. Nachdem das Reaktionsgemisch 30 Minuten bei -75° C weitergerührt wurde, wird eine Lösung von 172 mg 2-{2(S)-Azido-2-[4(S)-isopropyl-5-oxo-tetrahydrofuran-2(S)-yl]-ethyl}-3(S)-methyl-butyraldehyd in 2 mL Tetrahydrofuran tropfenweise zugefügt. Es wird nochmals 30 Minuten bei -75° C gerührt und dann bei der gleichen Temperatur 1.2 mL gesättigte Ammoniumchloridlösung zugetropft. Nachdem das Reaktionsgemisch auf Raumtemperatur gebracht wurde, wird dreimal mit Essigsäureethylester extrahiert. Aus den organischen Phasen erhält man nach Waschen mit Wasser (zweimal) und gesättigter Kochsalzlösung (einmal) , Trocknen über Magnesiumsulfat, Vereinigen, Eindampfen und Reinigen des Rückstandes mittels FC (zweimal 30 g Kieselgel, Hexan/Essigsäureethylester 6 : 2) die Titelverbindung: R_{f} (Hexan/Essigsäureethylester 2 : 1 ) = 0.23; HPLC Rₜ = 20.3 und 21.1 Minuten. (Diastereomerengemisch); FAB-MS M⁺ = 611.

### 2) {2(S)-{2(S)-Azido-2-[4(S)-isopropyl-5-oxo-tetrahydrofuran-2(S)-yl]-ethyl}-3-methyl-1(R,S)-[4-(3-benzyloxypropyl)-3-(3-methoxypropoxy)-phenyl]-butyl}-acetat

Eine Lösung von 144 mg 5(S)-{1(S)-Azido-3(S)-{(R,S)-hydroxy-[4-(3-benzyloxypropoxy)-3-(3-methoxypropoxy)-phenyl-methyl)-4-methyl -pentyl)-3(S)-isopropyl-tetrahydrofuran-2-on in 1.8 mL Essigsäureanhydrid und 0.057 mL Pyridin wird 30 Stunden bei Raumtemperatur gerührt und dann unter vermindertem Druck zur Trockne eingedampft. Der Rückstand wird zwischen Dichlormethan (dreimal) und Wasser-gesättigter Kochsalzlösung (dreimal) verteilt. Aus den organischen Phasen erhält man nach Vereinigen, Trocknen über Magnesiumsulfat, Eindampfen und Reinigen des Rückstandes mittels FC (Hexan/Essigsäureethylester 4:1) die Titelverbindung: R_{f} (Hexan/Essigsäureethylester 2:1) = 0.38 und 0.33; HPLC Rₜ = 21.8 und 21.8 Minuten. (Diastereomerengemisch); FAB-MS M+ = 653, (M+Na)⁺ = 676.

### 3) 5(S)-{1(S)-Amino-3(S)-[4-(3-hydroxypropoxy)-3-(3-methoxypropoxy)-benzyl]-4-methylpentyl}-3(S)-isopropyl-tetrahydrofuran-2-on

Eine Lösung von 151 mg {2(S)-{2(S)-Azido-2-[4(S)-isopropyl-5-oxo-tetrahydrofuran-2(S)-yl]-ethyl}-3-methyl-1 (R,S)-[4-(3-benzyloxypropoxy)-3-(3-methoxypropoxy)-phenyl]-butyl}-acetat in 10 mL Ethanol wird bei Normaldruck und Raumtemperatur in Gegenwart von 70 mg PdO 170 Stunden hydriert. Das Reaktionsgemisch wird filtriert, eingedampft und der Rückstand in 10 mL Ethanol gelöst und nochmals 24 Stunden in Gegenwart von 140 mg PdO bei Normaldruck und Raumtemperatur hydriert. Filtrieren und Eindampfen ergibt die Titelverbindung als Rohprodukt: R_{f} (Dichlormethan/Methanol) = 0.32; HPLC Rₜ = 11.7 Minuten.; FAB-MS (M+H)⁺ = 480. Die Verbindung wird ohne Reinigung in der nächsten Stufe eingesetzt.

### 4) 5(S)-{1(S)-(tert-Butoxycarbonyl)amino-3(S)-[4-(3-hydroxypropoxy)-3-(3-methoxypropoxy)-benzyl]-4-methyl-pentyl}-3(S)-isopropyl-tetrahydrofuran-2-on

Zu einer bei 0° C gerührten Lösung von 106 mg 5(S)-{1 (S)-Amino-3(S)-[4-(3-hydroxypropoxy)-3-(3-methoxypropoxy)-benzyl]-4-methyl-pentyl)-3(S)-isopropyl-tetrahydrofuran-2-on in 4.5 mL Dichlormethan wird die Lösung von 0.07 mL N-Ethyldiisopropylamin in 0.1 mL Dichlormethan und anschließend die Lösung von 77 mg Di-tertiärbutyl-dicarbonat in 0.4 mL Dichlormethan zugetropft. Das Reaktionsgemisch wird auf Raumtemperatur gebracht, 20 Stunden bei Raumtemperatur weitergerührt und dann zur Trockne eingedampft. Aus dem Rückstand erhält man durch Reinigung mittels FC (50 g Kieselgel, Dichlormethan/Methanol 98: 2) die Titelverbindung: R_{f} (Dichlormethan/Methanol 95 : 5) = 0.34; HPLC Rₜ = 19.1 Minuten.; FAB-MS M⁺ = 579, (M+Na)⁺ = 602.

### 5) 5(S)-(tert-Butoxycarbonyl)amino-4(S)-hydroxy-2(S)-isopropyl-7(S)-[4-(3-hydroxypropoxy)- 3-(3-methoxypropoxy)-benzyl]-8-methyl-nonansäure-N-(2-morpholin-4-yl-ethyl)amid

Ein Gemisch von 84 mg 5(S)-{1(S)-(*tert*-Butoxycarbonyl)amino-3(S)-[4-(3-hydroxypropoxy)-3-(3-methoxypropoxy)-benzyl]-4-methyl-pentyl}-3(S)-isopropyl-tetrahydrofuran-2-on, 0.6 mL 4-(2-Aminoethyl)-morpholin und 0.025 mL Eisessig wird 16 Stunden bei Raumtemperatur und 6 Stunden bei 45° C gerührt und dann zwischen Diethylether (zweimal) und gesättigter Natriumhydrogencarbonatlösung (einmal) und Wasser (zweimal) verteilt. Aus den organischen Phasen erhält man durch Vereinigen, Trocknen über Magnesiumsulfat, Eindampfen und Reinigen mittels FC (5 g Kieselgel, Dichlormethan/Methanol 98 : 2) die Titelverbindung: R_{f} (Dichlormethan/Methanol 95 : 5) = 0.16; HPLC Rₜ = 14.5 Minuten.; FAB-MS (M+H)⁺ = 710.

### 6) 5(S)-Amino-4(S)-hydroxy-2(S)-isopropyl-7(S)-[4-(3-hydroxypropoxy)-3-(3-methoxypropoxy)-benzyl]-8-methyl-nonansäure-N-(2-morpholin-4-yl-ethyl)amid-hydrochlorid

30 mg 5(S)-(*tert*-Butoxycarbonyl)amino-4(S)-hydroxy-2(S)-isopropyl-7(S)-[4-(3-hydroxypropoxy)-3-(3-methoxypropoxy)-benzyl]-8-methyl-nonansäure-N-(2-morpholin-4-yl-ethyl)amid werden in 1.5 mL einer auf 0° C gekühlten 4 N Lösung von Salzsäure in Dioxan gelöst und 10 Minuten. bei 0° C weitergerührt. Das Reaktionsgemisch wird unter vermindertem Druck bei Raumtemperatur zur Trockne eingedampft. Aus dem Rückstand erhält man durch Reinigen mittels FC (5 g Kieselgel, Dichlormethan -Methanol 98 : 2) die Titelverbindung: R_{f} (Dichlormethan/Methanol 8:2) = 0.20; Rₜ = 10.43 Minuten.; FAB-MS (M+H)⁺ = 610.

Das als Ausgangsmaterial verwendete 4-(3-Benzyloxypropoxy)-3-(3-methoxypropoxy)-brombenzol wird wie folgt hergestellt:

### a) 2-(3-Methoxypropoxy)-phenol

Eine Suspension von 8.4 g Natriumhydrid (60%ige Dispersion in Mineralöl) in 300 mL Dimethylformamid wird bei Raumtemperatur innert 30 Minuten. mit der Lösung von 22 g Brenzcatechin in 80 mL N,N-Dimethylformamid versetzt und 1 Stunde bei Raumtemperatur weitergerührt. Danach wird die Lösung von 49.3 g 3-Brompropyl-methylether in 80 mL Dimethylformamid zugetropft. Das Reaktionsgemisch wird 80 Stunden bei Raumtemperatur weitergerührt und dann unter vermindertem Druck bei 30° C Badtemperatur eingedampft. Der Rückstand wird zwischen Diethylether und Wasser verteilt. Aus den vereinigten organischen Phasen erhält man nach Trocknen über Magnesiumsulfat, Eindampfen und Reinigen des Rückstandes durch FC (100 g Kieselgel, Hexan -Dichlormethan 5: 95) die Titelverbindung: R_{f} (Dichlormethan/Diethylether 96 : 4) = 0.35; HPLC Rₜ = 11.2 Minuten.

### b) 4-Brom-2-(3-methoxypropoxy)-phenol

Eine Lösung von 2.6 g 2-(3-Methoxypropoxy)-phenol in 60 mL Dichlormethan und 40 mL Methanol wird bei Raumtemperatur mit 6.9 g Tetrabutylammoniumtribromid portionenweise versetzt und danach 30 Minuten weitergerührt. Das Reaktionsgemisch wird eingedampft und der Rückstand zwischen Diethylether und Wasser verteilt. Aus den vereinigten organischen Phasen erhält man nach Trocknen über Magnesiumsulfat, Eindampfen und Reinigen des Rückstandes durch FC (700 g Kieselgel, Dichlormethan/Diethylether 98 : 2) die Titelverbindung: R_{f} (Dichlormethan/Diethylether 97: 3) = 0.50; HPLC Rₜ = 14.3 Minuten; FAB-MS (M+H)⁺ = 262.

### c) 4-(3-Benzyloxypropoxy)-3-(3-methoxypropoxy)-brombenzol

Ein Gemisch von 4 g 4-Brom-2-(3-methoxypropoxy)-phenol, 2.3 g Kaliumcarbonat, 3.8 g Benzyl-(3-brompropyl)-ether, einer Spatelspitze Natriumjodid und 15 mL Acetonitril wird 30 Stunden unter Rückfluß gerührt. Das Reaktionsgemisch wird filtriert und das Filtrat eingedampft. Der Rückstand wird zwischen Essigsäureethylester und Wasser verteilt. Aus den organischen Phasen erhält man durch Vereinigen, Trocknen über Magnesiumsulfat, Eindampfen und Reinigen des Rückstandes mittels FC (Hexan/Essigsäureethylester 95 : 5) die Titelverbindung: R_{f} (Hexan/Essigsäureethylester = 9:1) = 0.15; HPLC Rₜ = 20.7 Minuten.

## Patentansprüche

1. Verbindungen der Formel I 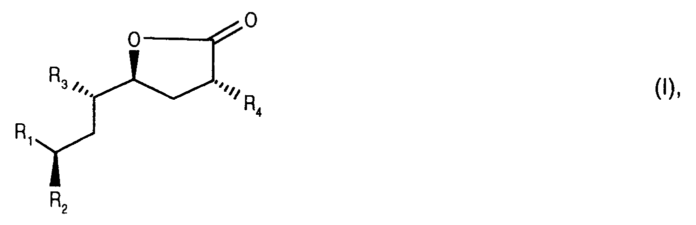 worin
R₁ gegebenenfalls verestertes Carboxy, Hydroxymethyl oder Formyl darstellt,
R₂ und R₄ unabhängig voneinander aliphatische, cycloaliphatisch-aliphatische, araliphatische oder heteroarylaliphatische Reste bedeuten und
R₃ Azido oder aliphatisch oder araliphatisch substituiertes und/oder durch eine Aminoschutzgruppe geschütztes Amino darstellt,
und ihre Salze.

2. Verbindungen gemäß Anspruch 1 der Formel I, worin
R₁ Carboxy, Niederalkoxycarbonyl, unsubstituiertes oder durch Niederalkyl, Niederalkoxy, Halogen und/oder Trifluormethyl substituiertes Phenylniederalkyoxycarbonyl, Hydroxymethyl oder Formyl darstellt,
R₂ und R₄ unabhängig voneinander Niederalkylreste, Niederalkenyl, Niederalkoxy oder Niederalkylthio, 3- bis 7-gliedrige Cycloalkylniederalkylreste, unsubstituierte oder durch Niederalkyl, Niederalkoxy, Hydroxy, Halogen, Nitro und/oder Amino substituierte Phenylniederalkyl- oder Naphthylniederalkylreste oder unsubstituiertes oder durch Niederalkyl, Niederalkoxy, Hydroxy, Halogen, Nitro und/oder Amino substituiertes Pyridylniederalkyl, Indolylniederalkyl, Chinolinylniederalkyl, Pyrimidinylniederalkyl, Furylniederalkyl, Benzofuranylniederalkyl oder Thienylniederalkyl bedeuten und
R₃ Azido, Niederalkylamino, Diniederalkylamino, Niederalkanoylamino oder N-Niederalkanoyl-N-niederalkyl-amino, unsubstituiertes oder durch Niederalkyl, Niederalkoxy, Hydroxy, Halogen, Nitro und/oder Amino substituiertes Phenylniederalkylamino, N-Niederalkyl-N-phenylniederalkyl-amino oder N-Niederalkanoyl-N-phenylniederalkyl-amino oder N-Niederalkoxycarbonylamino, N-Niederalkyl-N-niederalkoxycarbonyl-amino, N-Niederalkanoyl-N-niederalkoxycarbonyl-amino, N-Niederalkoxycarbonyl-N-phenylniederalkylamino, N-Phenylniederalkoxycarbonylamino, N- Niederalkyl-N-phenylniederalkoxycamonyl-amino, N-Niederalkanoyl-N-phenylniederalkoxycarbonyl-amino, N-Phenylniederalkoxycarbonyl-N-phenylniederalkylamino, N-Triniederalkylsilylamino, N-Niederalkyl-N-triniederalkylsilyl-amino, N-Niederalkanoyl-N-triniederalkylsilyl-amino oder N-Triniederalkylsilyl-N-phenylniederalkylamino darstellt,
und ihre Salze.

3. Verbindungen gemäß Anspruch 1 der Formel I, worin
R₁ Carboxy bedeutet,
R₂ C₁-C₄-Alkyl bedeutet,
R₄ C₁-C₄-Alkyl, C2-C4-Alkenyl oder unsubstituiertes oder durch C₁-C₄-Alkyl, C₁-C₄-Alkoxy, Hydroxy, Halogen der Atomnummer bis und mit 35, Nitro und/oder Amino substituiertes Phenyl-C₁-C₄-niederalkyl bedeutet und
R₃ Azido, C₁-C₄-Alkoxycarbonylamino oder Phenyl-C₁-C₄-Ikoxycarbonylamino ist,
und ihre Salze.

4. 2(S)-[2(S)-Azido-2(S)-(4(S)-isopropyl-5-oxo-tetrahydrofuran-2(S)-yl)-ethyl]-3-methyl-buttersäure oder ein Salz davon.

5. 2-{2(S)-Azido-2-[4(S)-isopropyl-5-oxo-tetrahydrofuran-2(S)-yl]-ethyl}-3(S)-methyl-butyraldehyd oder ein Salz davon.

6. 2-{2(S)-Azido-2-[4(S)-isopropyl-5-oxo-tetrahydrofuran-2(S)-yl]-ethyl}-3(S)-methyl-butanol oder ein Salz davon.

7. 3(S)-Isopropyl-5(S)-(1(S)-(*tert*-Butoxycarbonyl)amino)-3(S)-isopropyl-4-hydroxy-butyl)-tetrahydrofuran-2-on oder ein Salz davon.

8. Verfahren zur Herstellung von Verbindungen der Formel I 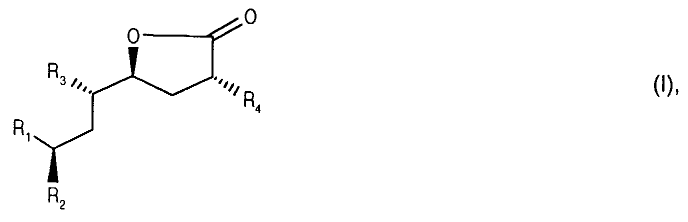 worin
R₁ gegebenenfalls verestertes Carboxy, Hydroxymethyl oder Formyl darstellt,
R₂ und R₄ unabhängig voneinander aliphatische, cycloaliphatisch-aliphatische, araliphatische oder heteroarylaliphatische Reste bedeuten und
R₃ Azido oder aliphatisch oder araliphatisch substituiertes und/oder durch eine Aminoschutzgruppe geschütztes Amino darstellt,
und ihre Salze, dadurch gekennzeichnet, daß man in einer Verbindung der Formel III 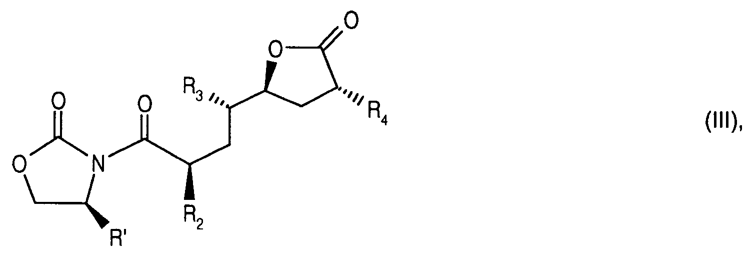 worin R' einen gegebenenfalls substituierten Kohlenwassertoffrest und R₃ beispielsweise Azido bedeutet, wobei R₂ und R₄ die angegebenen Bedeutungen haben, die 4-R'-2-oxo-oxazolidin-1-ylcarbonylgruppe selektiv zu Carboxy hydrolysiert, einen dabei gegebenenfalls geöffneten Laktonring unter Verwendung eines sauren Katalysators wieder schließt und gewünschtenfalls eine erfindungsgemäße Verbindung der Formel I in eine andere erfindungsgemäße Verbindung der Formel I umwandelt, ein gegebenenfalls erhältliche Isomerengemische auftrennt und/oder die verfahrensgemäß erhältliche Verbindung der Formel I in ein Salz oder ein verfahrensgemäß erhältliches Salz in die freie Verbindung oder in ein anderes Salz überführt.

9. Verbindungen der Formeln III, V und VI 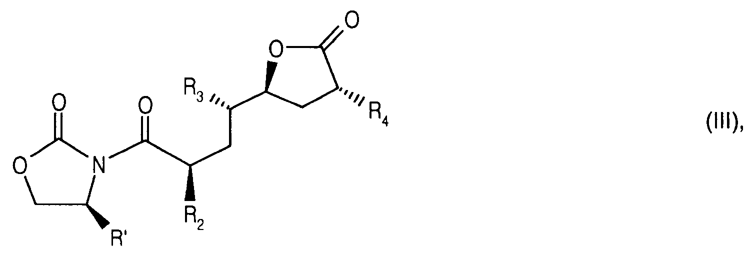 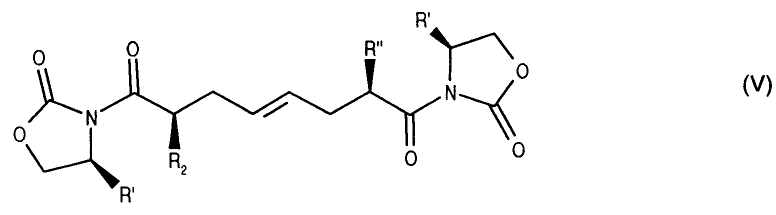 und 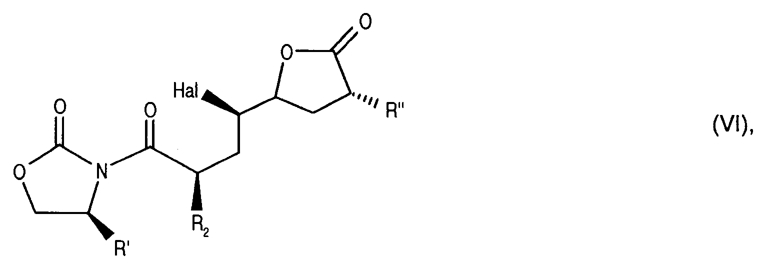 worin
R₂, R₄ und R'' unabhängig voneinander aliphatische, cycloaliphatisch-aliphatische, araliphatische oder heteroarylaliphatische Reste bedeuten,
R₃ Azido oder aliphatisch oder araliphatisch substituiertes und/oder durch eine Aminoschutzgruppe geschütztes Amino darstellt,
R' einen aliphatischen, cycloaliphatischen oder cycloaliphatisch-aliphatischen Kohlenwassertoffrest oder einen unsubstituierten oder durch Niederalkyl, Niederalkoxy, Halogen, Trifluormethyl, Nitro, Amino und/oder Mono- oder Diniederalkylamino substituierten araliphatische oder aromatische Kohlenwassertoffrest bedeutet und
Hal Halogen darstellt,
und ihre Salze.

10. Verbindungen gemäß Anspruch 9 der Formeln III, V und VI, worin
R₂, R₄ und R'' unabhängig voneinander Niederalkyl, Niederalkenyl, Niederalkoxy oder Niederalkylthio, 3- bis 7-gliedrige Cycloalkylniederalkylreste, unsubstituierte oder durch Niederalkyl, Niederalkoxy, Hydroxy, Halogen, Nitro und/oder Amino substituierte Phenylniederalkyl- oder Naphthylniederalkylreste oder unsubstituiertes oder durch Niederalkyl, Niederalkoxy, Hydroxy, Halogen, Nitro und/oder Amino substituiertes Pyridylniederalkyl, Indolylniederalkyl, Chinolinylniederalkyl, Pyrimidinylniederalkyl, Furylniederalkyl, Benzofuranylniederalkyl oder Thienylniederalkyl bedeuten,
R₃ Azido, Niederalkylamino, Diniederalkylamino, Niederalkanoylamino oder N-Niederalkanoyl-N-niederalkyl-amino, unsubstituiertes oder durch Niederalkyl, Niederalkoxy, Hydroxy, Halogen, Nitro und/oder Amino substituiertes Phenylniederalkylamino, N-Niederalkyl-N-phenylniederalkyl-amino oder N-Niederalkanoyl-N-phenylniederalkyl-amino oder N-Niederalkoxycarbonylamino, N-Niederalkyl-N-niederalkoxycarbonyl-amino, N-Niederalkanoyl-N-niederalkoxycarbonyl-amino, N-Niederalkoxycarbonyl-N-phenylniederalkylamino, N-Phenylniederalkoxycarbonylamino, N-Niederalkyl-N-phenyl-niederalkoxycarbonyl-amino, N-Niederalkanoyl-N-phenylniederalkoxycarbonyl-amino, N-Phenylniederalkoxycarbonyl-N-phenylniederalkylamino, N-Triniederalkylsilylamino, N-Niederalkyl-N-triniederalkylsilyl-amino, N-Niederalkanoyl-N-triniederalkylsilyl-amino oder N-Triniederalkylsilyl-N-phenylniederalkylamino darstellt und
R'' Niederalkyl, Niederalkenyl, 3- bis 5-gliedriges Cycloalkyl, 3- bis 5-gliedriges Cycloalkylniederalkyl, unsubstituiertes oder im Phenylteil durch Niederalkyl, Niederalkoxy, Halogen, Trifluormethyl, Nitro, Amino und/oder Mono- oder Diniederalkylamino substituiertes Phenylniederalkyl, unsubstituiertes oder im Naphthylteil durch Niederalkyl, Niederalkoxy, Halogen, Trifluormethyl, Nitro, Amino und/oder Mono- oder Diniederalkylamino substituiertes Naphthylniederalkyl oder unsubstituiertes oder durch Niederalkyl, Niederalkoxy, Halogen, Trifluormethyl, Nitro, Amino und/oder Mono- oder Diniederalkylamino substituiertes, Phenyl oder Naphthyl bedeutet,
und ihre Salze.

11. Verbindungen gemäß Anspruch 9 der Formel III, V oder VI, worin
R₂ und R₄ C₁-C₄-Alkyl, C₂-C₄-Alkenyl, wie Allyl oder Methallyl, C₁-C₅-Alkoxy, C₁-C₅-Alkylthio, 3- bis 7-gliedrige Cycloalkyl-C₁-C₄-alkyl, unsubstituiertes oder durch C₁-C₄-Alkyl, C₁-C₄-Alkoxy, Hydroxy, Halogen der Atomnummer bis und mit 35, Nitro und/oder Amino substituierte Phenyl-C₁-C₄-niederalkyl bedeutet,
R' unsubstituiertes oder durch C₁-C₄-Alkyl, C₁-C₄-Alkoxy, Hydroxy, Halogen der Atomnummer bis und mit 35, Nitro und/oder Amino substituierte Phenyl-C₁-C₄-niederalkyl ist,
Hal Halogen der Atomnummer bis und mit 35 darstellt und
R₃ Azido ist, und und ihre Salze.

12. Verbindungen gemäß Anspruch 9 der Formeln II, V oder VI, worin
R₂ C₁-C₄-Alkyl bedeutet,
R₄ und R'' C₁-C₄-Alkyl, C₂-C₄-Alkenyl oder unsubstituiertes oder durch C₁-C₄-Alkyl, C₁-C₄-Alkoxy, Hydroxy, Halogen der Atomnummer bis und mit 35, Nitro und/oder Amino substituiertes Phenyl-C₁-C₄-niederalkyl bedeutet,
R' Benzyl bedeutet,
Hal
Halogen der Atomnummer bis und mit 35 darstellt und
R₃ Azido ist,
und und ihre Salze.

13. 3-{2(S)-[2(S)-Azido-2(S)-(4(S)-isopropyl-5-oxo-tetrahydrofuran-2(S)-yl)-ethyl]-3-methyl-butyryl}-4(S)-benzyl-oxazolidin-2-on oder ein Salz davon.

14. 4(S)-Benzyl-3-{2(S)-[2(R)-brom-2(R)-(4(S)-isopropyl-5-oxo-tetrahydrofuran-2(S)-yl)-ethyl]-3-methyl-butyryl}-oxazolidin-2-on oder ein Salz davon.

15. 3-{2(S)-[2(S)-Azido-2(S)-(4(S)-isopropyl-5-oxo-tetrahydrofuran-2(S)-yl)-ethyl]-3-methylbutyryl}-4(S)-benzyl-oxazolidin-2-on oder ein Salz davon.

16. Verfahren zur Herstellung von Verbindungen der Formeln III, V und VI 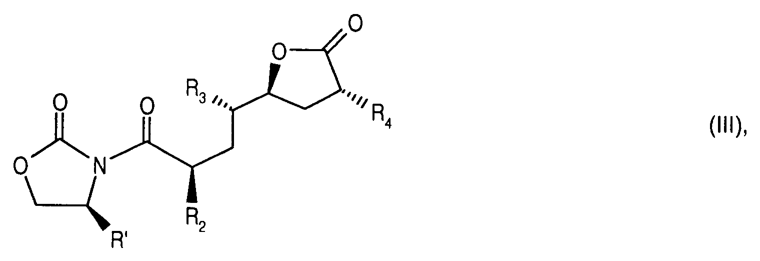 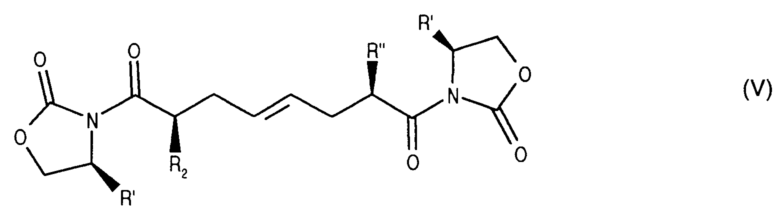 und 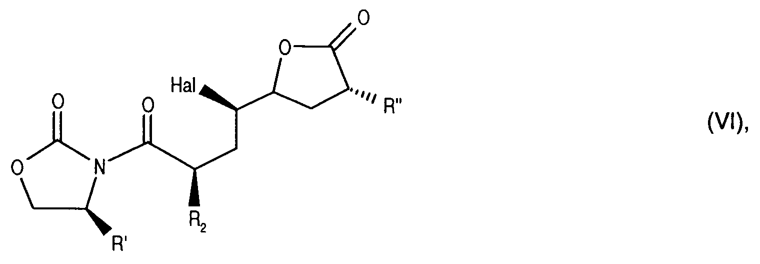 worin
R₂, R₄ und R'' unabhängig voneinander aliphatische, cycloaliphatisch-aliphatische, araliphatische oder heteroarylaliphatische Reste bedeuten,
R₃ Azido oder aliphatisch oder araliphatisch substituiertes und/oder durch eine Aminoschutzgruppe geschütztes Amino darstellt,
R' einen aliphatischen, cycloaliphatischen oder cycloaliphatisch-aliphatischen Kohlenwassertoffrest oder einen unsubstituierten oder durch Niederalkyl, Niederalkoxy, Halogen, Trifluormethyl, Nitro, Amino und/oder Mono- oder Diniederalkylamino substituierten araliphatische oder aromatische Kohlenwassertoffrest bedeutet und
Hal Halogen darstellt, und ihre Salze, dadurch gekennzeichnet, daß man
a) zur Herstellung von Verbindungen der Formel V ein 1,4-Dihalogenbuten, z.B. 1,4-Dibrombuten, zunächst mit einer Verbindung der Formel IVa und anschließend mit einer Verbindung der Formel IVb bzw. zunächst mit einer Verbindung der Formel IVb und anschließend mit einer Verbindung der Formel IVa 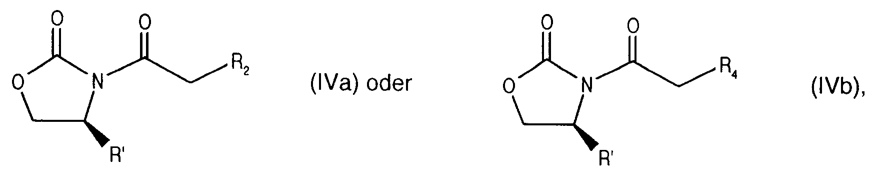 oder in einem Schritt oder schrittweise mit der etwa doppeltmolaren Menge einer Verbindung der Formel IVa oder IVb umsetzt, worin R', R₂ und R₄ die angegebenen Bedeutungen haben, oder
b) zur Herstellung von Verbindungen der Formel VI eine Verbindung der Formel V 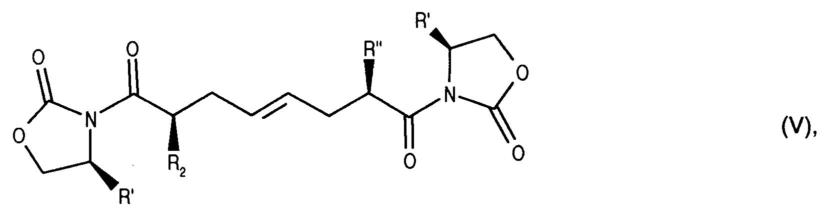 worin R'' für Reste R₂ oder R₄ steht, mit einem üblichen Halogenierungsmittel behandelt und das gewünschte Isomere, in dem R'' für R₄ steht, abtrennt und/oder
c) zur Herstellung von Verbindungen der Formel III in einer Verbindung der Formel VI 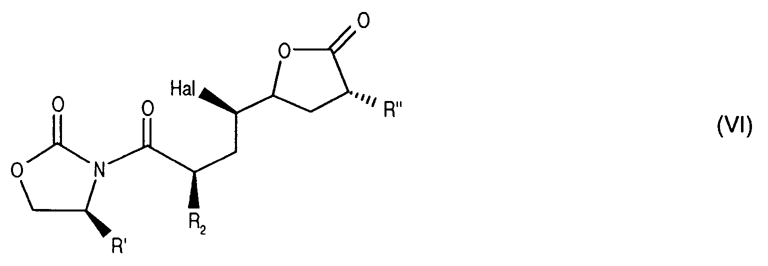 worin Hal Halogen bedeutet, das Halogenatom unter Inversion durch Azido ersetzt und gewünschtenfalls die Azidogruppe zu Amino hydriert und anschließend aliphatisch oder araliphatisch substituiert und/oder durch eine Aminoschutzgruppe schützt und gewünschtenfalls eine erfindungsgemäße Verbindung der Formeln III, V oder VI in eine andere erfindungsgemäße Verbindung der Formel Formeln III, V oder VI umwandelt, ein gegebenenfalls erhältliche Isomerengemische auftrennt und/oder die verfahrensgemäß erhältliche Verbindung der Formeln III, V oder VI in ein Salz oder ein verfahrensgemäß erhältliches Salz in die freie Verbindung oder in ein anderes Salz überführt.

17. Verwendung von Verbindungen der Formeln III, V, und/oder VI zur Herstellung von Verbindungen IIa und IIb 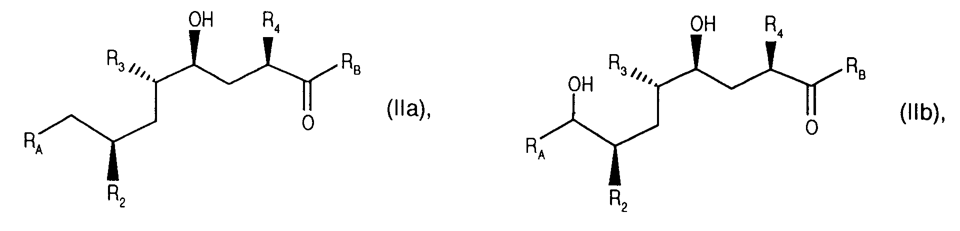 worin
R_{A} einen aromatischen oder heteroaromatischen Rest bedeutet,
R₂ und R₄ unabhängig voneinander aliphatische, cycloaliphatische, cycloaliphatisch-alipha tische oder araliphatische Reste darstellen,
R₃ für unsubstituiertes oder N-mono- oder N,N-diniederalkyliertes oder N-niederalkanoyliertes Amino steht und
R_{B} eine aliphatisch, cycloaliphatisch oder heteroaromatisch-aliphatisch substituierte Aminogruppe bedeutet,
und ihrer Salze, dadurch gekennzeichnet, daß man eine Verbindung der Formel I, worin R₁ für gegebenenfalls verestertes Carboxy steht, durch Reduktion oder eine Verbindung der Formel I, worin R₁ Hydroxymethyl bedeutet, durch Dehydrierung in den entsprechenden Aldehyd der Formel la 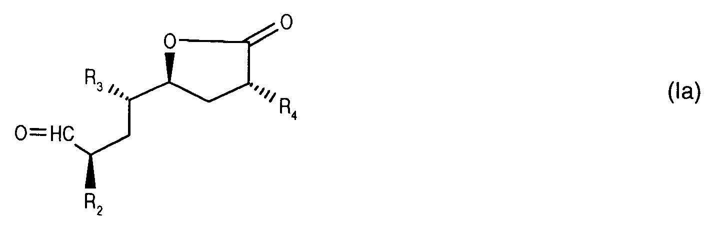 überführt, diesen mit einer Halogenmagnesiumverbindung der Formel R_{A}-Mg-Hal (Ic) kondensiert, die erhaltene Verbindungen der Formel Id 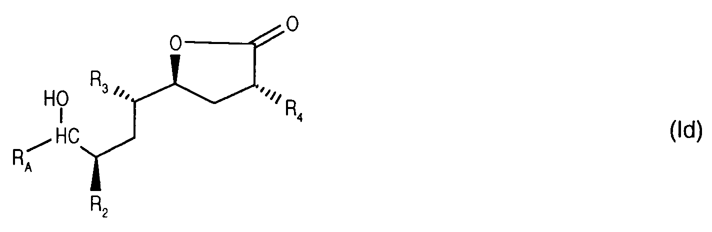 an der Hydroxygruppe intermediär schützt, die erhaltene Verbindung der Formel le 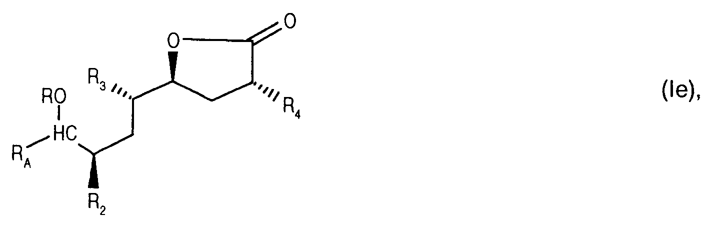 worin R eine Hydroxyschutzgruppe bedeutet, mit einem Amin der Formel H-R_{E} (If) umsetzt und die erhaltene Verbindung der Formel Ig 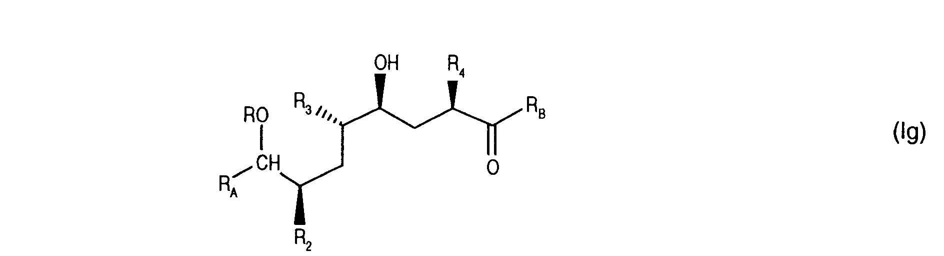 katalytisch hydriert, wobei die Gruppe R-O- durch Wasserstoff ersetzt sowie eine gegebenenfalls vorhandene Azidogruppe R₃ zu Amino reduziert und die entsprechende Verbindung der Formel II, worin R₃ für unsubstituiertes oder N-mono- oder N,N-diniederalkyliertes oder N-niederalkanoyliertes Amino steht, erhalten wird.
